# EUROPEAN PATENT SPECIFICATION

(11) **EP 2 395 085 B1**
(45) Date of publication and mention of the grant of the patent: **10.06.2015**
(21) Application number: 10738736.7
(22) Date of filing: 04.02.2010
(51) Int. Cl.: C12N 15/11, C12N 15/113

(54) **SMALL INTERFERENCE RNA COMPLEX WITH INCREASED INTRACELLULAR TRANSMISSION CAPACITY**
RNA-KOMPLEX MIT GERINGER INTERFERENZ UND ERHÖHTER INTRAZELLULÄRER ÜBERTRAGUNGSKAPAZITÄT
COMPLEXE DE PETIT ARN INTERFÉRENCE AVEC UNE CAPACITÉ DE TRANSMISSION INTRACELLULAIRE AUGMENTÉE

(30) Priority: 04.02.2009 KR 20090009036
(43) Date of publication of application: 14.12.2011
(73) Proprietor: Sungkyunkwan University Foundation for Corporate Collaboration, Gyeonggi-do 440-746 (KR)
(72) Inventor: LEE, Dong Ki, Seoul 137-776 (KR); CHANG, Chan Il, Daejeon 302-241 (KR)
(74) Representative: Hiebl, Inge Elisabeth
(86) International application number: PCT/KR2010/000678
(87) International publication number: WO 2010/090452

(56) References cited:
- WO-A2-2007/091269
- KR-A- 20070 114 923
- US-A1- 2004 147 476
- US-A1- 2005 196 781
- US-A1- 2006 178 327
- US-A1- 2007 254 362
- JI HOON JEONG ET AL: "siRNA conjugate delivery systems", BIOCONJUGATE CHEMISTRY, ACS, WASHINGTON, DC, US, vol. 20, no. 1, 21 January 2009 (2009-01-21), pages 5-14, XP002624571, ISSN: 1043-1802, DOI: 10.1021/BC800278E [retrieved on 2008-11-17]
- AMARSANAA JAZAG ET AL: 'Single small-interfering RNA expression vector for silencing multiple transforming growth factor-pathway components' NUCLEIC ACIDS RESEARCH vol. 33, no. 15, 2005, page E131, XP008152627
- CHAN IL CHANG ET AL: 'A structure-activity relationship study of siRNAs with structural variations' BIOCHEMICAL AND BIOPHYSICAL RESEARCH COMMUNICATIONS vol. 359, no. 4, 2007, pages 997 - 1003, XP022188405

<110> SUNGKYUNKWAN UNIVERSITY FOUNDATION FOR CORPORATE COLLABORATION
<120> Small Interfering RNA Complex Having Enhanced Intracellular Delivery
<130> PP-B0891
<150> KR10-2009-0009036
   <151> 2009-02-04
<160> 77
<170> KopatentIn 1.71
<210> 1
   <211> 38
   <212> RNA
   <213> Artificial Sequence
<220>
   <223> 1st strand for tsiRNA about Lamin A/C, DBP and TIG mRNAs
<400> 1
   uguucuucug gaaguccagu cgaagacauc gcuucuca 38
<210> 2
   <211> 38
   <212> RNA
   <213> Artificial Sequence
<220>
   <223> 2nd strand for tsiRNA about Lamin A/C, DBP and TIG mRNAs
<400> 2
   ugagaagcga ugucuucgac ugucucaggc guucucua 38
<210> 3
   <211> 38
   <212> RNA
   <213> Artificial Sequence
<220>
   <223> 3rd strand for tsiRNA about Lamin A/C, DBP and TIG mRNAs
<400> 3
   uagagaacgc cugagacagc uggacuucca gaagaaca 38
<210> 4
   <211> 21
   <212> DNA
   <213> Artificial Sequence
<220>
   <223> sense strand of siRNA for Lamin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 4
   cuggacuucc agaagaacat t 21
<210> 5
   <211> 21
   <212> DNA
   <213> Artificial Sequence
<220>
   <223> antisense strand of siRNA for Lamin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 5
   uguucuucug gaaguccagt t 21
<210> 6
   <211> 21
   <212> DNA
   <213> Artificial Sequence
<220>
   <223> sense strand of siRNA for DBP mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 6
   ucgaagacau cgcuucucat t 21
<210> 7
   <211> 21
   <212> DNA
   <213> Artificial Sequence
<220>
   <223> antisense strand of siRNA for DBP mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 7
   ugagaagcga ugucuucgat t 21
<210> 8
   <211> 21
   <212> DNA
   <213> Artificial Sequence
<220>
   <223> sense strand of siRNA for TIG3 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 8
   cugucucagg cguucucuat t 21
<210> 9
   <211> 21
   <212> DNA
   <213> Artificial Sequence
<220>
   <223> antisense strand of siRNA for TIG3 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 9
   uagagaacgc cugagacagt t 21
<210> 10
   <211> 20
   <212> DNA
   <213> Artificial Sequence
<220>
   <223> DBP-forward primer
<400> 10
   cctcgaagac atcgcttctc 20
<210> 11
   <211> 20
   <212> DNA
   <213> Artificial Sequence
<220>
   <223> DBP-reverse primer
<400> 11
   gcaccgatat ctggttctcc 20
<210> 12
   <211> 20
   <212> DNA
   <213> Artificial Sequence
<220>
   <223> GAPDH-forward primer
<400> 12
   gagtcaacgg atttggtcgt 20
<210> 13
   <211> 20
   <212> DNA
   <213> Artificial Sequence
<220>
   <223> GAPDH-reverse primer
<400> 13
   gacaagcttc ccgttctcag 20
<210> 14
   <211> 20
   <212> DNA
   <213> Artificial Sequence
<220>
   <223> Lamin-forward primer
<400> 14
   ccgagtctga agaggtggtc 20
<210> 15
   <211> 20
   <212> DNA
   <213> Artificial Sequence
<220>
   <223> Lamin-reverse primer
<400> 15
   aggtcaccct ccttcttggt 20
<210> 16
   <211> 20
   <212> DNA
   <213> Artificial Sequence
<220>
   <223> TIG3-forward primer
<400> 16
   agattttccg ccttggctat 20
<210> 17
   <211> 20
   <212> DNA
   <213> Artificial Sequence
<220>
   <223> TIG3-reverse primer
<400> 17
   tttcacctct gcactgttgc 20
<210> 18
   <211> 38
   <212> RNA
   <213> Artificial Sequence
<220>
   <223> 1st strand for tsiRNA-CVA
<400> 18
   ucaauacggu guuugcucuu ggugaugaug uaauugcu 38
<210> 19
   <211> 38
   <212> RNA
   <213> Artificial Sequence
<220>
   <223> 2nd strand for tsiRNA-CVA
<400> 19
   agcaauuaca ucaucaccac caugacucca gcugacaa 38
<210> 20
   <211> 38
   <212> RNA
   <213> Artificial Sequence
<220>
   <223> 3rd strand for tsiRNA-CVA
<400> 20
   uugucagcug gagucaugga gagcaaacac cguauuga 38
<210> 21
   <211> 21
   <212> DNA
   <213> Artificial Sequence
<220>
   <223> sense strand of siRNA for CVA-CRE mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 21
   agagcaaaca ccguauugat t 21
<210> 22
   <211> 21
   <212> DNA
   <213> Artificial Sequence
<220>
   <223> antisense strand of siRNA for CVA-CRE mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 22
   ucaauacggu guuugcucut t 21
<210> 23
   <211> 21
   <212> DNA
   <213> Artificial Sequence
<220>
   <223> sense strand of siRNA for CVA-3D1 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 23
   uggugaugau guaauugcut t 21
<210> 24
   <211> 21
   <212> DNA
   <213> Artificial Sequence
<220>
   <223> antisense strand of siRNA for CVA-3D1 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 24
   agcaauuaca ucaucaccat t
<210> 25
   <211> 21
   <212> DNA
   <213> Artificial Sequence
<220>
   <223> sense strand of siRNA for CVA-3D2 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 25
   ccaugacucc agcugacaat t 21
<210> 26
   <211> 21
   <212> DNA
   <213> Artificial Sequence
<220>
   <223> antisense strand of siRNA for CVA-3D2 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 26
   uugucagcug gagucauggt t 21
<210> 27
   <211> 25
   <212> DNA
   <213> Artificial Sequence
<220>
   <223> strand of target sequence of siCVA-CRE
<400> 27
   agctttcaat acggtgtttg ctcta 25
<210> 28
   <211> 25
   <212> DNA
   <213> Artificial Sequence
<220>
   <223> strand of traget sequence of siCVA-CRE
<400> 28
   ctagtagagc aaacaccgta ttgaa 25
<210> 29
   <211> 25
   <212> DNA
   <213> Artificial Sequence
<220>
   <223> strand of traget sequence of siCVA-3D1
<400> 29
   agcttagcaa ttacatcatc accaa 25
<210> 30
   <211> 25
   <212> DNA
   <213> Artificial Sequence
<220>
   <223> strand of traget sequence of siCVA-3D1
<400> 30
   ctagttggtg atgatgtaat tgcta 25
<210> 31
   <211> 25
   <212> DNA
   <213> Artificial Sequence
<220>
   <223> strand of traget sequence of siCVA-3D2
<400> 31
   agcttttgtc agctggagtc atgga 25
<210> 32
   <211> 25
   <212> DNA
   <213> Artificial Sequence
<220>
   <223> strand of traget sequence of siCVA-3D2
<400> 32
   ctagtccatg actccagctg acaaa 25
<210> 33
   <211> 25
   <212> DNA
   <213> Artificial Sequence
<220>
   <223> TIG3 Gene specific 3' primer
<400> 33
   ggggcagatg gctgtttatt gatcc 25
<210> 34
   <211> 25
   <212> DNA
   <213> Artificial Sequence
<220>
   <223> TIG3 Gene specific 3'nested primer
<400> 34
   acttttgcca gcgagagagg gaaac 25
<210> 35
   <211> 25
   <212> DNA
   <213> Artificial Sequence
<220>
   <223> Lamin Gene specific 3'primer
<400> 35
   ccagtgagtc ctccaggtct cgaag 25
<210> 36
   <211> 24
   <212> DNA
   <213> Artificial Sequence
<220>
   <223> Lamin Gene specific 3'nested primer
<400> 36
   cctggcattg tccagcttgg caga 24
<210> 37
   <211> 22
   <212> DNA
   <213> Artificial Sequence
<220>
   <223> DBP gene specific primer
<400> 37
   cgggacagca cggcgcggta gt 22
<210> 38
   <211> 23
   <212> DNA
   <213> Artificial Sequence
<220>
   <223> DBP gene specific 3'nested primer
<400> 38
   ctcctggcgc acggccacaa ctt 23
<210> 39
   <211> 17
   <212> DNA
   <213> Artificial Sequence
<220>
   <223> M13 forward primer
<400> 39
   gttttcccag tcacgac 17
<210> 40
   <211> 25
   <212> DNA
   <213> Artificial Sequence
<220>
   <223> IFN-beta forward primer
<400> 40
   agaagtctgc acctgaaaag atatt 25
<210> 41
   <211> 23
   <212> DNA
   <213> Artificial Sequence
<220>
   <223> IFN-beta reverse primer
<400> 41
   tgtactcctt ggccttcagg taa 23
<210> 42
   <211> 17
   <212> DNA
   <213> Artificial Sequence
<220>
   <223> DNA oligonucleotides linked to Trebler phosphoramidite
<400> 42
   aaccgtggtc atgctcc 17
<210> 43
   <211> 36
   <212> RNA
   <213> Artificial Sequence
<220>
   <223> RNA oligonucleotides having antisense strand of siSurvivin and complementary strand of DNA oligonucleotides of SEQ ID NO:42
<400> 43
   ugaaaauguu gaucuccuuu uggagcauga ccacgg 36
<210> 44
   <211> 21
   <212> DNA
   <213> Artificial Sequence
<220>
   <223> sense strand of siRNA for Survivin mRNA
<220>
   <223> molecule is combined RNA/DNA
<220>
   <221> misc_feature
   <222> (1)..(19)
   <223> ribonucleotides
<220>
   <221> misc_feature
   <222> (10)..(21)
   <223> deoxyribonucleotides
<400> 44
   aaggagauca acauuuucat t 21
<210> 45
   <211> 21
   <212> DNA
   <213> Artificial Sequence
<220>
   <223> antisense strand of siRNA for Survivin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 45
   ugaaaauguu gaucuccuut t 21
<210> 46
   <211> 21
   <212> DNA
   <213> Artificial Sequence
<220>
   <223> sense strand of siRNA for Survivin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 46
   aaggagauca acauuuucat t
<210> 47
   <211> 36
   <212> RNA
   <213> Artificial Sequence
<220>
   <223> antisense strand of long siSurvivin
<400> 47
   ugaaaauguu gaucuccuuu uggagcauga ccacgg 36
<210> 48
   <211> 20
   <212> DNA
   <213> Artificial Sequence
<220>
   <223> Survivin forward primer
<400> 48
   gcaccacttc cagggtttat 20
<210> 49
   <211> 20
   <212> DNA
   <213> Artificial Sequence
<220>
   <223> Survivin reverse primer
<400> 49
   ctctggtgcc actttcaaga 20
<210> 50
   <211> 39
   <212> RNA
   <213> Artificial Sequence
<220>
   <223> RNA oligonucleotides having antisense strand of siIntegrin and complementary strand of DNA oligonucleotides of SEQ ID NO:42
<400> 50
   ucaacaucag ucugauaagc uauuggagca ugaccacgg 39
<210> 51
   <211> 21
   <212> DNA
   <213> Artificial Sequence
<220>
   <223> sense strand of siRNA for Integrin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 51
   ugaacugcac uucagauaut t 21
<210> 52
   <211> 36
   <212> RNA
   <213> Artificial Sequence
<220>
   <223> RNA oligonucleotides having antisense strand of siBeta-catenin and complementary strand of DNA oligonucleotides of SEQ ID NO:42
<400> 52
   guccaucaau aucagcuacu uggagcauga ccacgg 36
<210> 53
   <211> 21
   <212> RNA
   <213> Artificial Sequence
<220>
   <223> sense strand of siRNA for Beta-catenin mRNA
<400> 53
   guagcugaua uugauggacu u 21
<210> 54
   <211> 20
   <212> DNA
   <213> Artificial Sequence
<220>
   <223> Integrin forward primer
<400> 54
   cgtatctgcg ggatgaatct 20
<210> 55
   <211> 20
   <212> DNA
   <213> Artificial Sequence
<220>
   <223> Integrin reverse primer
<400> 55
   gggttgcaag cctgttgtat 20
<210> 56
   <211> 20
   <212> DNA
   <213> Artificial Sequence
<220>
   <223> Beta-catenin forward primer
<400> 56
   atgtccagcg tttggctgaa 20
<210> 57
   <211> 20
   <212> DNA
   <213> Artificial Sequence
<220>
   <223> Beta-catenin reverse primer
<400> 57
   tggtcctcgt catttagcag 20
<210> 58
   <211> 21
   <212> DNA
   <213> Artificial Sequence
<220>
   <223> sense strand of siRNA for Integrin mRNA
<220>
   <223> molecule is combined RNA/DNA
<220>
   <221> misc_feature
   <222> (1)..(19)
   <223> ribonucleotides
<220>
   <221> misc_feature
   <222> (20).. (21)
   <223> deoxyribonucleotides
<400> 58
   ugaacugcac uucagauaut t 21
<210> 59
   <211> 21
   <212> DNA
   <213> Artificial Sequence
<220>
   <223> antisense strand of siRNA for Integrin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 59
   auaucugaag ugcaguucat t 21
<210> 60
   <211> 21
   <212> RNA
   <213> Artificial Sequence
<220>
   <223> sense strand of siRNA for Beta-catenin mRNA
<400> 60
   guagcugaua uugauggacu u 21
<210> 61
   <211> 21
   <212> RNA
   <213> Artificial Sequence
<220>
   <223> antisense strand of siRNA for Beta-catenin mRNA
<400> 61
   guccaucaau aucagcuacu u 21
<210> 62
   <211> 39
   <212> RNA
   <213> Artificial Sequence
<220>
   <223> RNA oligonucleotide having Anti-miR21 and complementary strand of DNA oligonucleotides of SEQ ID NO:42
<400> 62
   ucaacaucag ucugauaagc uauuggagca ugaccacgg 39
<210> 63
   <211> 51
   <212> DNA
   <213> Artificial Sequence
<220>
   <223> sense strand of target sequence of miR-21
<400> 63
   aatgcactag ttcaacatca gtctgataag ctagctcagc aagcttaatg c 51
<210> 64
   <211> 51
   <212> DNA
   <213> Artificial Sequence
<220>
   <223> Antisense strand of target sequence of miR-21
<400> 64
   gcattaagct tgctgagcta gcttatcaga ctgatgttga actagtgcat t 51
<210> 65
   <211> 22
   <212> RNA
   <213> Artificial Sequence
<220>
   <223> anti-miR21
<400> 65
   ucaacaucag ucugauaagc ua 22
<210> 66
   <211> 21
   <212> DNA
   <213> Artificial Sequence
<220>
   <223> IFIT1-Forward primer
<400> 66
   gccacaaaaa atcacaagcc a 21
<210> 67
   <211> 21
   <212> DNA
   <213> Artificial Sequence
<220>
   <223> IFIT1-reverse primer
<400> 67
   ccattgtctg gatttaagcg g 21
<210> 68
   <211> 21
   <212> DNA
   <213> Artificial Sequence
<220>
   <223> OAS2-forward primer
<400> 68
   tcagaagaga agccaacgtg a 21
<210> 69
   <211> 20
   <212> DNA
   <213> Artificial Sequence
<220>
   <223> OAS2-reverse primer
<400> 69
   cggagacagc gagggtaaat 20
<210> 70
   <211> 27
   <212> RNA
   <213> Artificial Sequence
<220>
   <223> Sense strand of 27bp dsRNA(Survivin)
<400> 70
   ucuuaggaaa ggagaucaac auuuuca 27
<210> 71
   <211> 27
   <212> RNA
   <213> Artificial Sequence
<220>
   <223> Antisense strand of 27bp dsRNA(Survivin)
<400> 71
   ugaaaauguu gaucuccuuu ccuaaga 27
<210> 72
   <211> 36
   <212> RNA
   <213> Artificial Sequence
<220>
   <223> mRNA sequence of Lamin A/C
<400> 72
   ggaacuggac uuccagaaga acaucuacag ugagga 36
<210> 73
   <211> 19
   <212> RNA
   <213> Artificial Sequence
<220>
   <223> Lamin antisense of tsiRNA-1
<400> 73
   uguucuucug gaaguccag 19
<210> 74
   <211> 36
   <212> RNA
   <213> Artificial Sequence
<220>
   <223> mRNA sequence of DBP
<400> 74
   acccucgaag acaucgcuuc ucagaagagg aacuua 36
<210> 75
   <211> 19
   <212> RNA
   <213> Artificial Sequence
<220>
   <223> DBP antisense of tsiRNA-1
<400> 75
   ugagaagcga ugucuucga 19
<210> 76
   <211> 36
   <212> RNA
   <213> Artificial Sequence
<220>
   <223> mRNA sequence of TIG3
<400> 76
   ugcccugucu caggcguucu cuagauccuu uccucu 36
<210> 77
   <211> 19
   <212> RNA
   <213> Artificial Sequence
<220>
   <223> TIG3 antisense of tsiRNA-1
<400> 77
   uagagaacgc cugagacag 19

## Description

### TECHNICAL FIELD

The present invention related to an siRNA complex and a multifunctional nucleic acid structure complex, which have enhanced intracellular delivery capacity.

### BACKGROUND ART

RNA interference (RNAi) is a mechanism capable of inhibiting the expression of a gene in a highly specific and efficient manner, in which degradation of the mRNA of a target gene is induced by introducing a double-stranded RNA (hereinafter referred to as "dsRNA"), which comprises a sense strand having a sequence homologous to the mRNA of the target gene and an antisense strand having a sequence complementary to the mRNA of the target gene, into cells or the like, thereby inhibiting the expression of the target gene.

With respect to these RNAi techniques, studies on modifications for increasing activity and/or intracellular delivery efficiency have been conducted so far. These RNAi techniques have been expected to be effective for the treatment of many diseases, including cancer or viral infection. Many researchers reported that the replication of viruses was successfully inhibited using various RNAi techniques, such as small interfering RNA (siRNA) and short hairpin RNA (shRNA) techniques (Jacque J.M. et al., Nature, 418: 435, 2002; Novina C.D. et al., Nat. Med., 8:681, 2002; Nishitsuji H. et al., Microbes Infect., 6:76, 2004). However, it was reported that, when the expression of a single target gene in a virus is inhibited by the RNAi mechanism, the virus can escape from RNAi-mediated inhibition of gene expression due to the substitution or deletion of a single nucleotide in the genome of the virus, (Westerhout E.M. et al., Nucleic Acids Res., 33:796, 2005). One way to minimize the escape of viruses from RNAi-mediated inhibition of gene expression is to induce multiple RNAi mechanisms that target various regions in the viral genome.

Meanwhile, RNAi mechanisms have been used to develop drugs not only for inhibiting viral replication, but also for treating cancer. RNAi can induce cell cycle arrest and target genes essential for tumor survival, thereby inducing apoptosis in cancer cells. It was reported that the simultaneous inhibition of a plurality of genes induces strong apoptosis in cancer cells (Menendez J.A. et al., Proc. Natl. Acad. Sci. U.S.A., 101:10715, 2004). Accordingly, the development of an efficient strategy for inhibiting the expression of a plurality of target genes using an RNAi mechanism has been required.

However, so far, multi-targeted RNAi mechanisms have been developed mainly based on shRNA expression systems (Konstantinova P. et al., Gene Ther., 13:1403, 2006; ter Brake O. et al., Mol. Ther., 14:883, 2006; Watanabe.T. et al., Gene Ther., 13:883, 200,6)). Also, Khaled et al. developed RNA nanostructures for non-viral siRNA delivery, which comprise multiple siRNAs based on phi29 RNA backbones (Khaled A. et al., Nano Lett., 5:1797, 2005). However, such RNA backbone structures have an excessively long length so that they cannot be chemically synthesized, and thus the actual use thereof is limited. Accordingly, it has been required to develop new siRNA structures which can be applied to multiple siRNAs and, at the same time, can be chemically synthesized.

Meanwhile, another requirement for the siRNA technique is the efficient intracellular delivery of siRNA. It was reported that 21-base-pair siRNAs known in the prior art is unsuitable for binding to a cationic polymer such as PEI, unlike plasmid DNAs (Balcato-Bellemin A.L. et al., Proc. Natl. Acad. Sci. U.S.A., 104:16050, 2007).

Accordingly, the present inventors have made extensive efforts to provide a novel siRNA complex which can inhibit the expression of a plurality of genes while having enhanced intracellular delivery capacity, and as a result, have found that a complex obtained by constructing a multiplex siRNA structure and a multifunctional nucleic acid structure and combining each of the structures with a cationic cell delivery vehicle effectively inhibits the expression of a plurality of genes and, at the same time, has significantly enhanced intracellular delivery capacity, thereby completing the present invention.

WO 2007/091269 discloses tandem siRNAs comprising consecutive nucleotides, wherein a first segment of such nucleotides encodes a first inhibitory RNA molecule, a second segement of such nucleotides encodes a second inhibitory RNA molecule and a third segment of such nucleotides encodes a third inhibitory RNA molecule.

US 2005/1996781 relates to compounds, compositions and methods useful for modulating STAT 3 gene expression using short-interfering nucleic acid (siNA) molecules, and also to compounds, compositions and methods useful for modulating the expression and activity of other genes involved in pathways of STAT3 gene expression and/or activity by RNA interference (RNAi) using small nucleic acid molecules.

### DISCLOSURE OF INVENTION

It is an object of the present invention to provide a novel siRNA complex which can inhibit the expression of a plurality of genes and, at the same time, has enhanced intracellular delivery capacity.

Another object of the present invention is to provide a novel multifunctional nucleic acid structure complex having enhanced intracellular delivery capacity.

To achieve the above objects, the present invention provides an siRNA complex having enhanced intracellular delivery capacity, in which a cationic cell delivery vehicle is bound to a multiplex siRNA structure comprising three siRNAs linked to each other at one end thereof, wherein each of said siRNAs is linked to another siRNA without a linker.

The present invention also provides the use of the siRNA complex in an in vitro method of delivering siRNA into cells, wherein said delivering comprises introducing said siRNA complex into cells.

The present invention also provides a multifunctional nucleic acid structure in which one end of each of three nucleic acid oligonucleotides having a cationic cell delivery vehicle bound to the oligonucleotides, selected from the group consisting of siRNAs, miRNAs, antagomiRs, antisense oligonucleotides, ribozymes and aptamers is linked to a compound having three functional groups, wherein the compound having three functional groups is any one of phosphoramidite compounds, iodoacetyl compounds, maleimide compounds, epoxide compounds, thiol-disulfide compounds, thiolated Ellman's reagent, NHS or sulfo-NHS compounds and isocyanate compounds;
wherein a nucleic acid fragment is linked to each functional group of the compound having three functional groups, and each of the nucleic acid oligonucleotides is linked to the nucleic acid fragment as a complementary bond by introducing a sequence, which can complementarily bind to the nucleic acid fragment, into any one among any one strand of sense or antisense strand of the siRNAs, miRNAs, antagomiRs, antisense oligonucleotides, ribozymes and aptamers.

The present invention also provides a multifunctional nucleic acid structure complex having enhanced intracellular delivery capacity, in which a cationic cell delivery vehicle is bound to said multifunctional nucleic acid structure.

The present invention also provides the use of the complex in an in vitro method of delivering a multifunctional nucleic acid structure into cells, wherein said delivery comprises introducing said multifunctional nucleic acid structure complex into cells.

Further disclosed is a method for preparing a multifunctional nucleic acid structure, the method comprising linking one end of each of nucleic acid oligonucleotides, selected from the group consisting of siRNAs, miRNAs, antagomiRs, antisense oligonucleotides, ribozymes and aptamers, to a compound having three or more functional groups.

The present invention also provides a method for preparing a multifunctional nucleic acid structure complex having enhanced intracellular delivery capacity, the method comprising the steps of: linking one end of each of nucleic acid oligonucleotides, selected from the group consisting of siRNAs, miRNAs, antagomiRs, antisense oligonucleotides, ribozymes and aptamers, to a compound having three functional groups, wherein the compound having three functional groups is anyone of phosphoramidite compounds, iodoacetyl compounds, maleimide compounds, epoxide compounds, thiol-disulfide compounds, thiolated Ellman's reagent, NHS or sulfo-NHS compounds and isocyanate compounds, thereby preparing a multi-functional nucleic acid structure, wherein a nucleic acid fragment is linked to each functional group of the compound having three functional groups, and each of the nucleic acid oligonucleotides is linked to the nucleic acid fragment as a complementary bond by introducing a sequence, which can complementarity bind to the nucleic acid fragment, into any one among any one strand of sense or antisense strand of the siRNAs, miRNAs, antagomiRs, antisense oligonucleotides, ribozymes and aptamers; and binding a cationic cell delivery vehicle to the multifunctional nucleic acid structure by charge-charge interaction.

Further disclosed is a composition for inhibiting gene expression containing said siRNA complex.

The present invention also provides the use of said siRNA complex for inhibition of gene expression.

Further disclosed is a method for inhibiting gene expression, the method comprising introducing said siRNA complex into cells.

Further disclosed is an anticancer composition containing said siRNA complex.

Further disclosed is the use of said siRNA complex for anticancer therapy.

Further disclosed is a method for inhibiting or treating cancer, the method comprising introducing said siRNA complex into cells.

Further disclosed is a composition for inhibiting gene expression containing said multifunctional nucleic acid structure complex.

Further disclosed is the use of said multifunctional nucleic acid structure complex for inhibition of gene expression.

Further disclosed is a method for inhibiting gene expression, the method comprising introducing said multifunctional nucleic acid structure complex into cells.

Further disclosed is an anticancer composition containing said multifunctional nucleic acid structure complex.

Further disclosed is the use of said multifunctional nucleic acid structure complex for anticancer therapy.

Further disclosed is a method for inhibiting or treating cancer, the method comprising introducing said multifunctional nucleic acid structure complex into cells.

Further disclosed is a kit for inhibiting gene expression comprising said composition for inhibiting gene expression.

Further disclosed is the use of said siRNA complex or multifunctional nucleic acid structure complex as an agent for treating viral infection.

Other features and embodiments of the present invention will be more apparent from the following detailed descriptions and the appended claims.

### BRIEF DESCRIPTION OF THE DRAWINGS

FIG. 1 shows the structure of the tsiRNA (multiplex siRNA structure) of an siRNA complex according to the present invention (FIG. 1a) and is a set of graphs showing the gene silencing activity of the siRNA complex of the present invention (FIGS. 1b to 1d).
FIG. 2 is a set of graphs showing the gene silencing activity of a multiplex siRNA structure bound to PEI (FIG. 2a) or Lipofectamine 2000 (FIG. 2b) according to the present invention.
FIG. 3 is a set of fluorescence microphotographs of HeLa cells introduced with each of an FITC-labeled siRNA mixture (siLamin, siDBP, and FITC-siTIG3) bound to PEI and an FITC-labeled tsiRNA (FITC-tsiRNA) bound to PEI.
FIG. 4 shows the inventive multiplex siRNA structure (tsiRNA-CVA) targeting different regions of the CVA24 genome (FIG. 4a) and is a set of graphs showing a comparison of relative luciferase activities measured after three siRNA mixtures targeting said regions have been introduced into cells using PEI together with three respective luciferase vectors comprising different viral nucleotide sequences.
FIG. 5 shows the results of carrying out 5'RACE analysis in order to confirm whether the siRNA structure according to the present invention inhibits gene expression by the same RNAi mechanism as that of 19+2 siRNA according to the prior art.
FIG. 6 is a graph showing a comparison between the levels of induction of interferon-β (IFN-β), measured by an RT-PCR after introducing an siRNA mixture and tsiRNA, which were each bound to PEI, in order to confirm whether the siRNA structure according to the present invention causes a nonspecific antiviral response.
FIG. 7 shows the structure of Trebler tsiSurvivin (T-tiSurvivin) used in the present invention.
FIG. 8 shows the nucleotide sequences of siSurvivin and long siSurvivin used as control groups for Trebler tsiSurvivin.
FIG. 9 is a set of graphs showing the gene silencing efficiency of a T-tiSurvivin-PEI delivery vehicle.
FIG. 10 shows a T-tiRNA structure prepared by binding siSurvivin, siIntegrin and siβ-catenin to Trebler phosphoramidite (FIG. 10a) and is a graph showing the gene silencing efficiency of the T-tiRNA structure (FIG. 10b).
FIG. 11 shows an MT-tiRNA structure prepared by binding Anti-miR21, siIntegrin and siβ-catenin to Trebler phosphoramidite (FIG. 11a) and is a set of graphs showing the gene silencing efficiency of the MT-tiRNA structure (FIG. 11b).
FIG. 12 is a graph showing a comparison of luciferase activities resulting from the introduction of T-tiAnti-miR21.
FIG. 13 shows the results of electrophoresis carried out to examine the rate of the isolation of a T-tiSurvivin caused by HS (FIG. 13a), a photograph showing the results of measuring intracellular delivery capacity using FITC (FIG.. 13b), and a set of graphs showing fluorescence intensity using flow cytometry (FIGS. 13c and 13d).
FIG. 14 shows a graph showing the gene silencing efficiency of a T-tiSurvivin complex (FIG. 14a), a graph showing the inhibition of growth of HepG2 cells after introduction of the T-tiSurvivin complex (FIG. 14b), and a set of microscope images (FIG. 14c).
FIG. 15 is a set of graphs showing the levels of expression of *IFITI* mRNA (a), *IFN*-β mRNA (b) and *OAS2* mRNA in T98G cells transfected with a tiRNA complex.
FIG. 16 shows the results of electrophoresis of 27-bp dsRNA, siLamin, tsiRNA, siSurvivin and T-tiSurvivin before and after treatment with Dicer.
FIG. 17 shows a structure obtained by chemical modification of some nucleotides of tsiRNA.
FIG. 18 shows a structure obtained by chemical modification of some nucleotides of T-tiSurvivin.
FIG. 19 shows the results of electrophoresis of tsiRNA(OMe) (a) and T-tiSurvivin(OMe) (b), each having a chemical modification, after Dicer treatment.
FIG. 20 is a set of graphs showing the gene silencing efficiencies of tsiRNA(OMe) (a) and T-tiSurvivin(OMe) (b), each having a chemical modification.

### BEST MODE FOR CARRYING OUT THE INVENTION

Unless otherwise defined, all technical and scientific terms used herein have the same meaning as commonly understood by one of ordinary skill in the art. Generally, the nomenclature used herein are well known and conventionally used in the art.

The definition of main terms used in the detailed description of the invention is as follows.

As used herein, the term "siRNA (small interfering RNA)" means a short double-stranded RNA (dsRNA) that mediates efficient gene silencing in a sequence-specific manner.

As used herein, the term "gene" is intended to have the broadest meaning, and the gene can encode a structural protein or a regulatory protein. Herein, the regulatory protein includes a transcriptional factor, a heat shock proteins, or a protein that is involved in DNA/RNA replication, transcription and/or translation. Also, the target gene whose expression is to be inhibited is resident in a viral genome which has integrated into the animal gene or may be present as an extrachromosomal element. For example, the target gene may be a gene on an HIV genome. In this case, the genetic construct is useful in inactivating translation of the HIV gene in a mammalian cell.

As used herein, the term "complex" of the multiplex siRNA or the multifunctional nucleic acid structure with the cationic cell delivery vehicle refers to a complex formed by a strong charge-charge interaction between the negative charge of the backbone of the multiplex siRNA and the positive charge of the cationic cell vehicle.

In one aspect, the present invention is directed to an siRNA complex having enhanced intracellular delivery capacity, in which a cationic cell delivery vehicle is bound to a multiplex siRNA structure comprising three siRNAs linked to each other at one end thereof, wherein each of said siRNAs is linked to another siRNA without a linker.

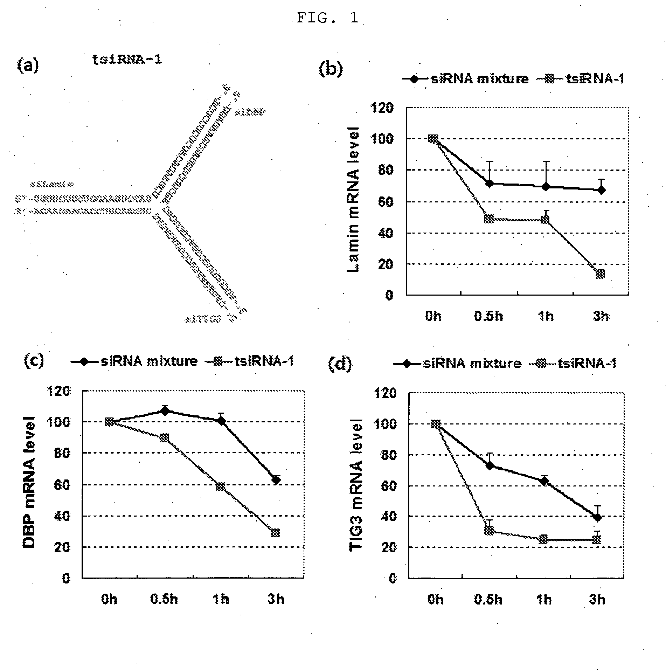

As used herein, the term "multiple siRNA" refers to an siRNA structure prepared in such a manner that three siRNAs are linked to each other at one end thereof. Three different siRNAs are linked to each other in order to inhibit the expression of a plurality of target genes while significantly increasing the intracellular delivery capacity thereof. Also, the multiplex siRNA preferably has a structure in which siRNAs are linked to each other at the 3'end of the antisense strand in such a manner that the 5' end of the antisense strand faces the outside. FIG. 1a shows the multiplex siRNA of the present invention, obtained by linking siRNAs to each other, which target three different target genes.

The siRNAs may comprise a chemical modification. For example, the chemical modification may be one in which the hydroxyl group at the 2' position of the ribose of at least one nucleotide included in the siRNAs was replaced by any one of a hydrogen atom, a fluorine atom, an -O-alkyl group, an - O-acyl group and an amino group.

Also, in the present invention, the cationic cell delivery vehicle is a positively charged delivery reagent which is used to deliver nucleic acid (i.e., siRNA) into cells under *in vitro* or *in vivo* conditions. The cationic cell delivery vehicle strongly interacts with the multiplex siRNA structure of the present invention to form a complex so that the siRNAs can be effectively introduced into cells. The cationic cell delivery vehicle that is used in the present invention may be made of a cationic polymer or a cationic lipid. For example, liposomes such as polyethylenimine (PEI) or Lipofectamine 2000 (Invitrogen) may be used as the cell delivery vehicle, but it will be obvious to a person skilled in the art that any positively charged delivery reagent may be used to provide the complex of the present invention.

In another aspect, the present invention is also directed to a multifunctional nucleic acid structure in which one end of each of three nucleic acid oligonucleotides having a cationic cell delivery vehicle bound to the oligonucleotides, selected from the group consisting of siRNAs, miRNAs, antagomiRs, antisense oligonucleotides, ribozymes and aptamers is linked to a compound having three functional groups, wherein the compound having three functional groups is any one of phosphoramidite compounds, iodoacetyl compounds, maleimide compounds, epoxide compounds, thiol-disulfide compounds, thiolated Ellman's reagent, NHS or sulfo-NHS compounds and isocyanate compounds;
wherein a nucleic acid fragment is linked to each functional group of the compound having three functional groups, and each of the nucleic acid oligonucleotides is linked to the nucleic acid fragment as a complementary bond by introducing a sequence, which can complementarily bind to the nucleic acid fragment, into any one among any one strand of sense or antisense strand of the siRNAs, miRNAs, antagomiRs, antisense oligonucleotides, ribozymes and aptamers.

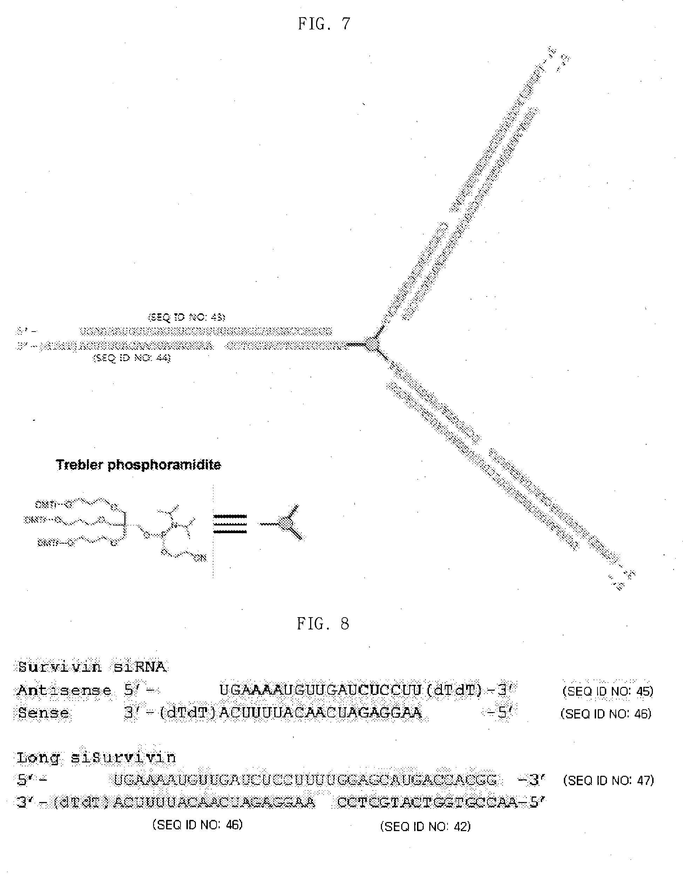

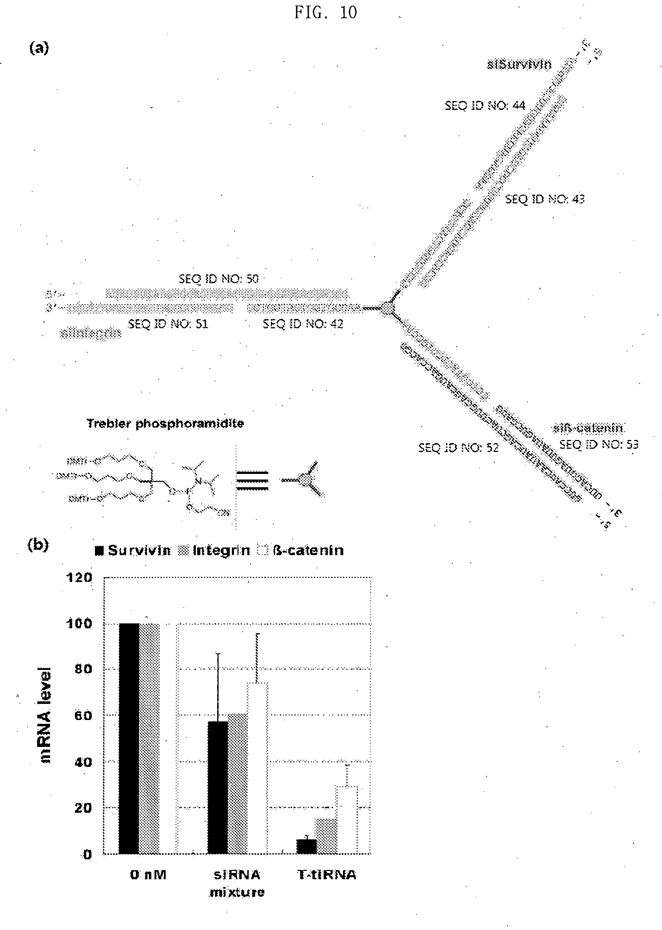

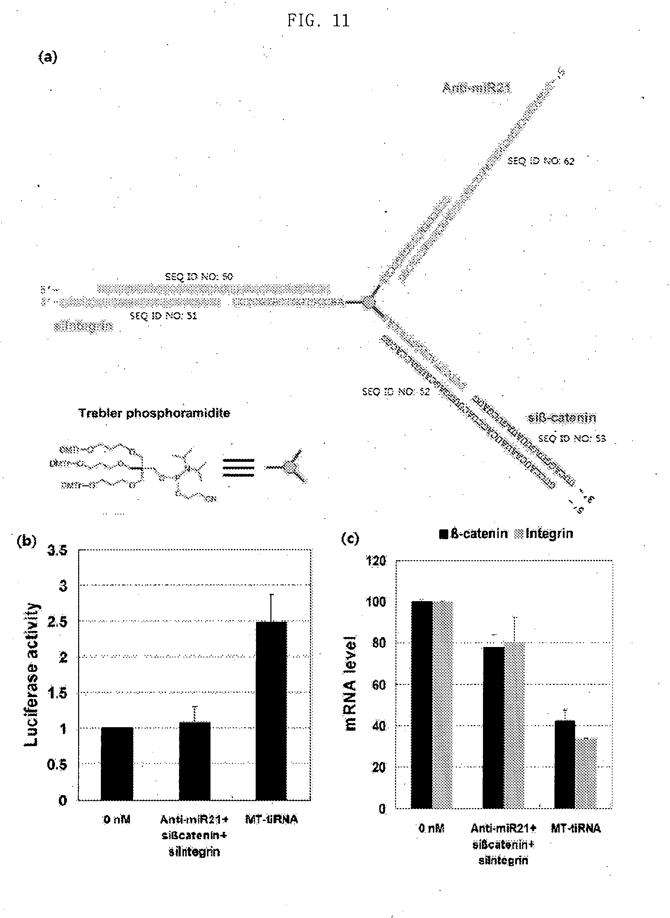

As used herein, the term "multifunctional nucleic acid structure" refers to a nucleic acid structure in which functional nucleic acid oligonucleotides such as siRNA, miRNA, antagomiR, an antisense oligonucleotide, ribozyme and an aptamer are bound to each functional group of a compound having three functional groups. As used herein, the term "three nucleic acid oligonucleotides" is intended to mean a structure either consisting of three nucleic acid oligonucleotides, or consisting of two or three identical nucleic acid oligonucleotides and one nucleic acid oligonucleotide different therefrom, or consisting of different nucleic acid nucleonucleotides. Specifically, as shown in FIGS. 7 and 10a, the structure may consist only of three or more siRNAs, or as shown in FIG. 11a, may consist of two s-iRNAs and one antagomiR.

These nucleic acid oligonucleotides may be prepared to comprise a chemical modification. For example, the chemical modification may be one in which the hydroxyl group at the 2' position of the ribose of at least one nucleotide included in the siRNA was replaced by any one of a hydrogen atom, a fluorine atom, an -O-alkyl group, an -O-acyl group and an amino group.

This multifunctional nucleic acid structure is prepared by binding one end of each of nucleic acid oligonucleotides, selected from the group consisting of siRNAs, miRNAs, antagomiRs, antisense, antisense oligonucleotides, ribozymes and aptamers, to a compound having three functional groups. Specifically, it can be prepared by linking a nucleic acid fragment to each functional group of a compound having three functional groups, and then introducing a sequence, which can complementarily bind to the nucleic acid fragment, into the nucleic acid oligonucleotide so as to complementarily bind to the nucleic acid fragment.

Example of the compound having three functional groups, which is used in the present invention, include, but are not limited to, phosphoramidite compounds, iodoacetyl compounds, maleimide compounds, epoxide compounds, thiol-disulfide compounds, thiolated Ellman's reagent, NHS or sulfo-NHS compounds, isocyanate compounds and the like. It will be obvious to a person skilled in the art that any which can bind to three siRNAs or other functional nucleic acid oligonucleotides can be used without any particular limitation as the compound having three functional groups. Herein, the compound having three functional groups can be suitably linked with oligonucleotides having a terminal substituent such as amine or thiol.

For example, where a phosphoramidite compound is used as the linker, a novel multifunctional nucleic acid structure can be constructed by linking three nucleic acid fragments to a phosphoramidite compound having three arms, such as Trebler phosphoramidite (Trilink Bio Technology), constructing siRNAs whose sense or antisense strand comprises a sequence capable of complementarily binding to the nucleic acid fragments, and linking the constructed siRNAs to the nucleic acid fragments by complementary binding. As used herein, the term "complementary binding" means that some fragments which are included in the sense or antisense strand of siRNAs have a complementarity of about 70-80% or greater, preferably about 80-90% or greater, and more preferably about 90-95% or greater, and still more preferably about 95-99%, with the sequences of the nucleic acid fragments, or can completely complementarily hybridize with the sequence of the nucleic acid fragments. However, a mismatch can be intentionally introduced into a region for linkage at a level in which annealing is possible.

According to the present invention, a multifunctional nucleic acid structure complex can be provided by binding a cationic cell delivery vehicle to the multifunctional nucleic acid structure of the present invention by charge-charge interaction. Accordingly, in another aspect, the present invention relates to a multifunctional nucleic acid structure complex having enhanced intracellular delivery capacity, which comprises a cationic cell delivery vehicle bound thereto.

The cationic cell delivery vehicle that is used in the present invention may be made of a cationic polymer or a cationic lipid. For example, liposomes such as polyethylenimine (PEI) or Lipofectamine 2000 (Invitrogen) may be used as the cationic cell delivery vehicle, but it will be obvious to a person skilled in the art that any positively charged delivery reagent may be used to provide the complex of the present invention.

In one Example of the present invention, each of the siRNA complex according to the present invention and an siRNA-cationic lipid complex according to the prior art was introduced into cells, and the gene silencing efficiency of each structure was measured. As a result, it was found that the siRNA complex according to the present invention inhibited the expression of all the target genes in a more efficient manner than the conventional siRNA structure.

In addition, in the case of treating viral infection by the RNAi mechanism according to the prior art, viral escape caused by the substitution or deletion of nucleotides in the viral genome has become a problem. However, in another Example of the present invention, multiple siRNAs targeting three different regions of the viral genome were introduced into cells using PEI, and as a result, it was found that the multiplex siRNA structure significantly reduced the expression of mRNAs including the corresponding viral genome regions, suggesting that the present invention is also useful for the treatment of viral infection.

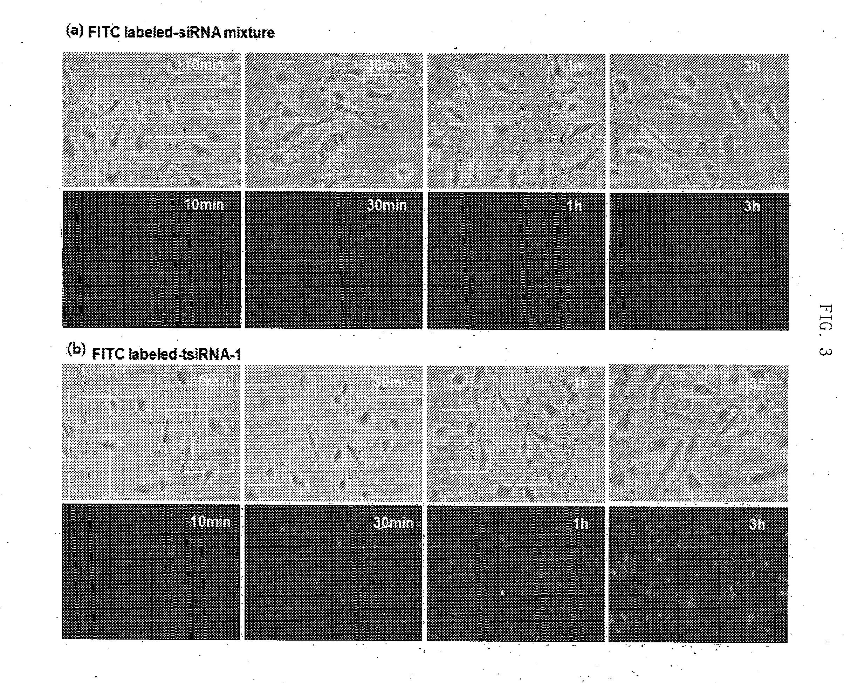

In still another Example of the present invention, the intracellular permeability of the siRNA complex was compared with conventional siRNAs using a fluorescence microscope. As a result, it was found that the siRNA complex according to the present invention had very excellent intracellular delivery capacity compared to the conventional siRNA structure bound to PEI. Specifically, the conventional siRNAs were reported to be unsuitable for binding to a cationic lipid such as polyethylenimine (PEI) and were found to have low intracellular permeability (FIG. 3), but it was found that the multiplex siRNA complex of the present invention did bind to a cationic cell delivery vehicle such as polyethylenimine (PEI), thereby exhibiting very high intracellular delivery capacity.

Accordingly, the present invention relates to the use of the siRNA complex in an in vitro method of delivering siRNA into cells, wherein said delivering comprises a step of introducing the siRNA complex of the present invention into cells.

In addition, in still another Example of the present invention, the mechanism of the siRNA complex according to the present invention was compared with the conventional siRNA structure in order to confirm whether the siRNA complex of the present invention can be used in the same applications as those for general RNAi mechanisms. As a result, it was found that the multiplex siRNA structure of the siRNA complex according to the present invention inhibited gene expression by the same mechanism as that of the 19+2 siRNA structure of the prior art. This suggests that the siRNA complex according to the present invention can be used in the same applications as those for conventional RNAi.

Accordingly, intracellular delivery of the siRNA complex can efficiently inhibit the expression of target genes. Thus, in another aspect, further disclosed is a composition for inhibiting gene expression containing an siRNA complex in which a cationic cell delivery complex is bound to a multiplex siRNA structure comprising three siRNAs linked to each other at one end thereof without a linker. Also, the present invention can provide a composition for inhibiting gene expression, which is in the form of a multifunctional nucleic acid structure complex comprising a cationic cell delivery vehicle bound to a multifunctional nucleic acid structure in which three siRNAs are linked with each other at one end thereof without a linker by a compound having three functional groups.

The multifunctional nucleic acid structure complex according to the present invention has a remarkable gene silencing effect, like the multiplex siRNA complex. Also, according to the present invention, the multifunctional nucleic acid structure may comprise, in addition to siRNAs, functional nucleic acid oligonucleotides, such as miRNA, antagomiR, an antisense oligonucleotide, an aptamer and ribozyme. Unlike multiple siRNAs that require a specific structure and the linkage of double-stranded RNAs such as siRNAs, a backbone structure having short oligonucleotides is constructed using a compound having three or more functional groups, whereby any nucleic acid oligonucleotide can be introduced into the structure. Thus, binding of single-stranded nucleic acid oligonucleotides such as antagomiR also becomes possible. In addition, because oligonucleotides are bound to the compound having three or more functional groups, a structure comprising nucleic acid fragments having a shorter length than multiple tsiRNAs can be provided, and thus the production cost of the structure is reduced and the preparation process thereof is also easily carried out.

In addition, in one Example of the present invention, whether the multifunctional nucleic acid structure complex and the multiplex siRNA complex according to the present invention induce immune responses was examined. As a result., it was found that the multiplex siRNA complex caused some nonspecific immune responses in T98G cells reported to have high immune sensitivity to dsRNA treatment, whereas the multifunctional nucleic acid structure complex did not cause such immune responses.

Also, in another Example of the present invention, resistance to Dicer was analyzed. As a result, it was found that the multifunctional nucleic acid structure complex showed higher resistance to Dicer.

Meanwhile, the composition for inhibiting gene expression may be provided in the form of a kit for inhibiting gene expression. The kit for inhibiting gene expression may take the form of bottles, tubs, sachets, envelops, tubes, ampoules, and the like, which may be formed in part or in whole from plastic, glass, paper, foil, wax, and the like. The container may be equipped with a fully or partially detachable lid that may initially be part of the container or may be affixed to the container by mechanical, adhesive, or other means. The container may also be equipped with a stopper, allowing access to the contents by a syringe needle. The kit may comprise an exterior package which may include instructions regarding the use of the components.

Also, the use of the composition for inhibiting gene expression can effectively inhibit the expression of two or more genes at the same time. Thus, in another aspect, further disclosed is a method of inhibiting gene expression using the composition for inhibiting gene expression.

In addition, in another Example of the present invention, it was found that the complex according to the present invention had the effect of inhibiting the growth of cancer cells. Thus, in still another aspect, further disclosed is an anticancer composition containing the multiplex siRNA complex or the multifunctional nucleic acid structure complex.

The anticancer composition may be provided as a pharmaceutical composition comprising the complex alone or in combination with at least one pharmaceutically acceptable carrier, excipient or diuent. The complex may be contained in the pharmaceutical composition in a pharmaceutically effective amount according to a disease and the severity thereof, the patient's age, weight, health condition and sex, the route of administration and the period of treatment.

As used herein, the term "pharmaceutically acceptable" refers to a composition that is physiologically acceptable and does not cause gastric disorder, allergic reactions such as gastrointestinal disorder or vertigo, or similar reactions, when administered to humans. Examples of said carrier, excipient or diluent may include lactose, dextrose, sucrose, sorbitol, mannitol, xylitol, erythritol, maltitol, starch, acacia rubber, alginate, gelatin, calcium phosphate, calcium silicate, cellulose, methyl cellulose, polyvinylpyrrolidone, water, methylhydroxybenzoate, propylhydroxybenzoate, talc, magnesium stearate and mineral oils.

The pharmaceutical composition may additionally contain fillers, anti-aggregating agents, lubricants, wetting agents, perfumes, emulsifiers and preservatives. Also, the pharmaceutical composition may be formulated using a method well known in the art, such that it can provide the rapid, sustained or delayed release of the active ingredient after administration to mammals. The formulation may be in the form of sterile injection solutions, etc.

### EXAMPLES

Hereinafter, the present invention will be described in further detail with reference to examples. It will be obvious to a person having ordinary skill in the art that these examples are illustrative purposes only and are not to be construed to limit the scope of the present invention.

Particularly, Lamin A/C, DBP, TIG3, Survivin gene and a CVA24 viral genome were illustrated as target genes in the following Examples, but it will be obvious to a person skilled in the art that the inventive complex and composition that target other genes can show the same results as those in the Examples. Also, it will be obvious to a person skilled in the art that the use of functional nucleic acid oligonucleotides other than AntimiR-21 used in the following. Examples can also provide the multifunctional nucleic acid structure of the present invention.

### Example 1: Preparation of siRNA complex according to the present invention

Multiple siRNAs used in Examples and siRNAs used in experiments were provided by purchasing chemically synthesized RNAs from Bioneer, Inc., and then annealing the RNAs according to the manufacturer's protocol.

First, a triple-target gene silencing siRNA (tsiRNA) targeting Lamin A/C, DBP and TIG3 mRNA were prepared as shown in FIG. 1a.

Three strands for providing the tsiRNA were as follows:
1^{st} strand: 5'-UGUUCUUCUGGAAGUCCAGUCGAAGACAUCGCUUCLTCA-3' (SEQ ID NO: 1)
2^{nd} strand: 5'-UGAGAAGCGAUGUCUUCGACUGUCUCAGGCGUUCUCUA-3' (SEQ ID NO: 2)
3^{rd} strand: 5'-UAGAGAACGCCUGAGACAGCUGGACUUCCAGAAGAACA-3' (SEQ ID NO: 3)

Meanwhile, siRNAs for the three regions, used as control groups, were as follows:
siRNA (siLamin) for Lamin mRNA
sense: 5'-CUGGACUUCCAGAAGAACA(dTdT)-3' (SEQ ID NO: 4)
antisense: 5'-UGUUCUUCUGGAAGUCCAG(dTdT)-3' (SEQ ID NO: 5)
siRNA (siDBP) for DBP mRNA
sense: 5'-UCGAAGACAUCGCUUCUCA(dTdT)-3' (SEQ ID NO: 6)
antisense: 5'-UGAGAAGCGAUGUCUUCGA(dTdT)-3' (SEQ ID NO: 7)
siRNA (siTIG3) for TIG3 mRNA
sense: 5'-CUGUCUCAGGCGUUCUCUA(dTdT)-3' (SEQ ID NO: 8)
antisense: 5'-UAGAGAACGCCUGAGACAG(dTdT)-3' (SEQ ID NO: 9)

To the tsiRNA obtained as described above, polyethylenimine (PEI; Polyplus) was added according to the manufacturer's protocol, thereby preparing an siRNA complex according to the present invention.

### Example 2: Measurement of gene silencing activities of siRNA complex according to the present invention and siRNAs according to the prior art

Each of the complex of PEI with the triple-target gene silencing siRNA (tsiRNA) targeting A/C (Lamin A/C), DBP and TIG3 mRNA, prepared in Example 1, and a complex of PEI with each of siLamin, siDBP and siTIG3 that are the conventional siRNA structures described in Example 1, was introduced into HeLa cells (ATCC CCL-2). The HeLa cells were cultured in Dulbecco's modified Eagle's medium (Hyclone), supplemented with 10% FBS (fetal bovine serum), in a 12-well plate. Before introduction of each complex, the cells were cultured in antibiotic-free complete medium for 24 hours until a confluency of 80% was reached. The siRNA mixture was used at a concentration of 100 nM, and the tsiRNA was also used at a concentration of 100 nM. The PEI (N/P=5) used was purchased from Polyplus and introduced according to the manufacturer's protocol.

3 hours after introduction of each complex, the medium was replaced with a fresh medium, and the levels of Lamin, DBP and TIG3 mRNA were measured by quantitative real-time RT-PCR. Specifically, total RNA was extracted from the cell lysate using a Tri-reagent kit (Ambion) and then used as a template for cDNA synthesis which was carried out using the ImProm-II™ reverse transcription system (Promega) according to the manufacturer's protocol. Then, the expression level of each of the target genes was measured using a step-one real-time PCR system (Applied Biosystems) according to the manufacturer's protocol, and the measured mRNA levels of Lamin, DBP and TIG3 were compared with the mRNA level of GAPDH (control group). Primers for each gene, used in the PCR amplification, were as follows:

### Primer pair for RT-PCR of DBP

DBP-forward 5'-CCT CGA AGA CAT CGC TTC TC-3' (SEQ ID NO: 10)
DBP-reverse 5'-GCA CCG ATA TCT GGT TCT CC-3' (SEQ ID NO: 11)

### Primer pair for RT-PCR of GAPDH

GAPDH-forward 5'-GAG TCA ACG GAT TTG GTC GT-3' (SEQ ID NO: 12)
GAPDH-reverse 5'-GAC AAG CTT CCC GTT CTC AG-3' (SEQ ID NO: 13)

### Primer pair for RT-PCR of Lamin

Lamin-forward 5'-CCG AGT CTG AAG AGG TGG TC-3' (SEQ ID NO: 14)
Lamin-reverse 5'-AGG TCA CCC TCC TTC TTG GT-3' (SEQ ID NO: 15)

### Primer pair for RT-PCR of TIG3

TIG3-forward 5'-AGA TTT TCC GCC TTG GCT AT-3' (SEQ ID NO: 16)
TIG3-reverse 5'-TTT CAC CTC TGC ACT GTT GC-3' (SEQ ID NO: 17)

After 30 minutes, 1 hour and 3 hours after introduction of each complex, the mRNA level of each of Lamin, DBP and TIG3 was measured. As a result, as shown in FIGs. 1b to 1d, the siRNA complex (tsiRNA) according to the present invention showed a more excellent gene silencing activity at all the time points.

Meanwhile, each of the siRNA complex (TsiRNA), obtained in Example 1, and an siRNA complex (TsiRNA-1) prepared by adding Lipofectamine 2000 (Invitrogen) to the siRNA complex (TsiRNA) of Example 1 according to the manufacturer's protocol, was introduced into HeLa cells (ATCC CCL-2) according to the same method as described above. 24 hours after introduction of each complex, the mRNA level of each gene was measured by RT-PCR.

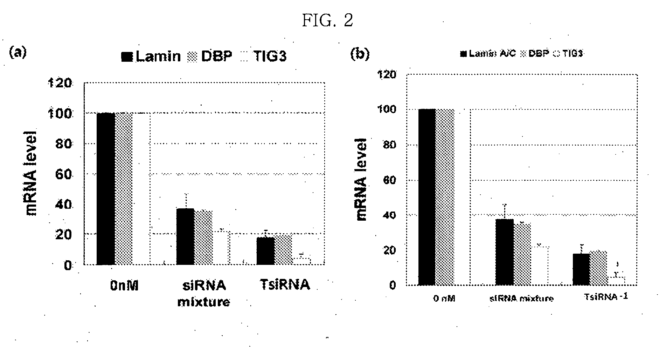

As a result, as can be seen in FIG. 2, the mixture of each siRNA with PEI showed very limited gene silencing activity, similar to prior reports (Bolcato-Bellemin A.L. et al., Proc. Natl. Acad. Sci. U.S.A., 104:16050, 2007), but in the case of the TsiRNA bound to the cationic polymer PEI, and the TsiRNA bound to the cationic lipid Lipofectamine 2000, the mRNA levels of the three genes were significantly low.

Such results indicate that the siRNA complex according to the present invention is effective in inhibiting the expression of the three different genes, in comparison with the conventional siRNA structure bound to the cationic cell delivery vehicle such as PEI or Lipofectamine 2000.

### Example 3: Measurement of intracellular delivery capacity of siRNA complex according to the present invention

In order to compare the intracellular delivery capacity between the siRNA complex of the present invention and the conventional siRNA (siTIG3), the following experiment was carried out.

The 3' end of the sense strand of siTIG3 and the 3' end of the TIG3 sense strand of tsiRNA were labeled with FITC. Also, each of the FITC-labeled siRNA mixture (siLamin, siDBP, and FITC-siTIG3) and the FITC-labeled tsiRNA (FITC-tsiRNA) was bound to PEI and introduced into HeLa cells. Then, the HeLa cells were observed with a fluorescence microscope (Olympus) at various time points.

The cells were observed 10 minutes, 30 minutes, 1 hour and 3 hours after introduction. As a result, as can be seen in FIG. 3, in the case in which the siRNA mixture was introduced using PEI (FIG. 3a), little or no fluorescence was observed at all the time points. However, in the case in which the FITC-labeled tsiRNA was introduced using PEI (FIG. 3b), green spots scattered in the intracellular regions could be observed, and the intensity of the spots was gradually increased as the culture time was increased.

Such results indicate that the siRNA complex according to the present invention has very high intracellular delivery capacity compared to the conventional siRNA structure bound to PEI.

### Example 4: Targeting of viral genome

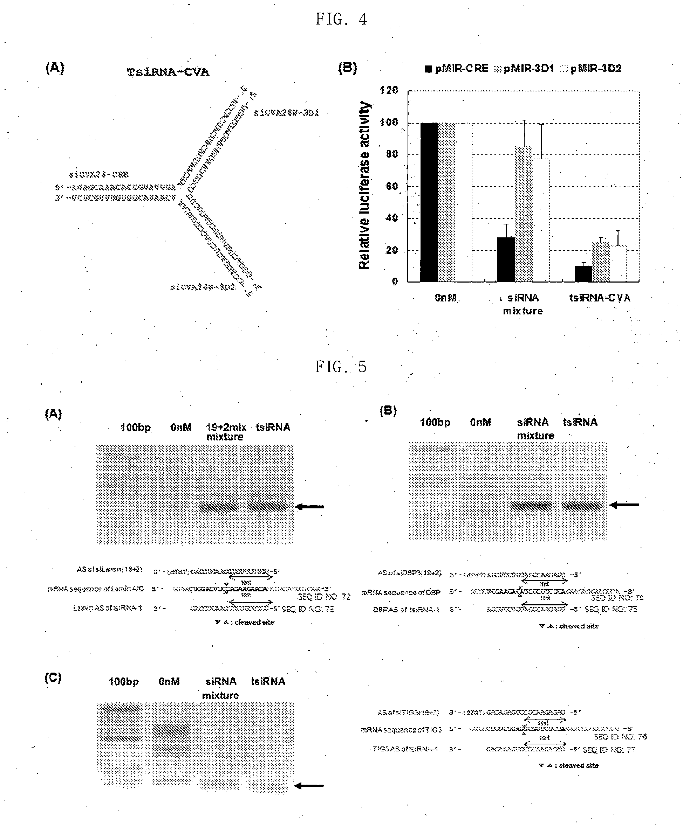

The inhibition of viral replication using the prior RNAi mechanism had a problem associated with a high rate of viral escape. In order to confirm whether this problem can be overcome and whether the siRNA structure according to the present invention is a general siRNA mechanism, the following experiments were carried out. For this purpose, the siRNA complex according to the present invention was prepared as shown in FIG. 4a (tsiRNA-CVA) so as to target three different regions (CRE, 3D1, and 3D2) of a viral genome (coxsackievirus A24; CVA24).

Three strands for providing the tsiRNA-CVA were as follows:
tsiRNA-CVA:
1^{st} strand: 5'-UCAAUACGGUGUUUGCUCUUGGUGAUGAUGUAAUUGCU-3' (SEQ ID NO: 18)
2^{nd} strand: 5'-AGCAAUUACAUCAUCACCACCAUGACUCCAGCUGACAA-3' (SEQ ID NO: 19)
3^{rd} strand: 5'-UUGUCAGCUGGAGUCAUGGAGAGCAAACACCGUAUUGA-3' (SEQ ID NO: 20)

Meanwhile, siRNAs for the three regions, used as control groups, were as follows:
siRNA (siCVA-CRE) for CVA-CRE mRNA
sense: 5'-AGAGCAAACACCGUAUUGA(dTdT)-3' (SEQ ID NO: 21)
antisense: 5'-UCAAUACGGUGUUUGCUCU(dTdT)-3' (SEQ ID NO: 22)
siRNA (siCVA-3D1) for CVA-3D1 mRNA
sense: 5'-UGGUGAUGAUGUAAUUGCU(dTdT)-3' (SEQ ID NO: 23)
antisense: 5'-AGCAAUUACAUCAUCACCA(dTdT)-3' (SEQ ID NO: 24)
siRNA (siCVA-3D2) for CVA-3D2 mRNA
sense: 5'-CCAUGACUCCAGCUGACAA(dTdT)-3' (SEQ ID NO: 25)
antisense: 5'-UUGUCAGCUGGAGUCAUGG(dTdT)-3' (SEQ ID NO: 26)

Then, each of a target sequence for siCVA-CRE, a target sequence for siCVA-3D1 and a target sequence for siCVA-3D2 was inserted into the SpeII and HindIII positions of the 3' untranslated region of the luciferase mRNA-encoding gene of a pMIR Report-luciferase vector (Ambion), thereby constructing pMIR-CRE, pMIR-3D1 and pMIR-3D2 vectors. The inserted target sequences were as follows:
CRE AS-target
Hind 5'-AGCTT T CAA TAC GGT GTT TGC TCT A -3'(SEQ ID NO: 27)
Spe 3' - A A GTT ATG CCA CAA ACG AGA TGATC-5' ( SEQ ID NO: 28)
3D1 AS-target
Hind 5'-AGCTT A GCA ATT ACA TCA TCA CCA A -3' (SEQ ID NO: 29)
Spe 3'- A T CGT TAA TGT AGT AGT GGT TGATC-5' (SEQ ID NO: 30)
3D2 AS-target
Hind 5' -AGCTT T TGT: CAG CTG GAG TCA TGG A 3' (SEQ ID NO: 31)
Spe 3' - A A ACA GTC GAC CTC AGT ACC TGATC-5' (SEQ ID NO: 32)

Each of the vectors was introduced into the complex of siRNA mixtures (siCVA-CRE, siCVA-3D1, and siCVA-3D2) with PEI and the complex of tsiRNA-CVA with PEI, and then introduced into HeLa cells, after which the luciferase activity of the cells was measured. Specifically, 24 hours after introduction of each vector, the cells were lysed using the passive lysis buffer of a dual-luciferase reporter assay system (Promega). The luciferase activity was measured using a Victor3 plate reader (PerkinElmer) for firefly and *Renilla* luciferase.

As a result, as can be seen in FIG. 4b, the siRNA complex according to the present invention showed significantly low luciferase activity compared to the complex of the conventional art siRNA structure bound to PEI, like the results of Example 2.

Such experimental results indicate that the siRNA complex according to the present invention is also useful for inhibition of viral replication showing a high escape rate for prior RNAi mechanisms, suggesting that the structure of the siRNA complex according to the present invention is not limited only to the target gene silencing of Example 2, but can be provided as a general siRNA mechanism having improved intracellular delivery capacity and gene silencing efficiency.

In addition, in order to confirm whether the siRNA complex according to the present invention can be used in the same applications as those for the prior RNAi mechanism, the following experiment on the analysis of mechanism was carried out.

### Example 5: Analysis of gene silencing mechanism

In order to confirm whether the siRNA structure according to the present invention inhibits gene expression by the same RNAi mechanism as that of the conventional 19+2 siRNA, the following experiment was carried out. Specifically, to analyze the cleavage site of each target mRNA, 5'RACE (Rapid amplification of cDNA ends) analysis was carried out.

First, the siRNA (siLamin, siDBP, and siTIG3) or the tsiRNA constructed in Example 1 was introduced into HeLa cells using PEI, and after 18 hours, total RNA was extracted from the cells using a Tri-reagent kit (Ambion). The total RNA (3 µg) was ligated with 0.25 µg of GeneRacer RNA oligo without pretreatment, and the GeneRacer RNA oligo-ligated total RNA was subjected to reverse transcription using GeneRacer oligo dT and a SuperScript^{™}III RT kit (Invitrogen). The RNA oligo-ligated mRNA was amplified using gene-specific primers. Next, the PCR product was cloned into a T&A vector (RBC), and then sequenced using an M13 forward primer.
TIG3 Gene specific 3'primer:
5'-GGGGCAGATGGCTGTTTATTGATCC-3'(SEQ ID NO: 33)
TIG3 Gene specific 3'nested primer:
5'-ACTTTTGCCAGCGAGAGAGGGAAAC-3'(SEQ ID NO: 34)
Lamin Gene specific 3'primer:
5'-CCAGTGAGTCCTCCAGGTCTCGAAG-3'(SEQ ID NO: 35)
Lamin Gene specific 3'nested primer:
5'-CCTGGCATTGTCCAGCTTGGCAGA-3'(SEQ ID NO: 36)
DBP gene specific primer
5'-CGGGACAGCACGGCGCGGTAGT-3'(SEQ ID NO: 37)
DBP gene specific 3' nested primer
5'-CTCCTGGCGCACGGCCACAACTT-3'(SEQ ID NO: 38)
M13 forward primer
5'-GTTTTCCCAGTCACGAC-3' (SEQ ID NO: 39)

As a result, as can be seen in FIG. 5, in both the case of treatment with the siRNA-PEI complex and the case of treatment with the tsiRNA-PEI complex, Lamin, DBP and TIG3 mRNA were digested at the same position.

Such experimental results indicate that the multiplex siRNA structure of the siRNA complex according to the present invention inhibits gene expression by the same mechanism as that of the conventional 19+2 siRNA structure.

### Example 6: Measurement of the induction of nonspecific antiviral responses

It was reported an RNA double strand having a length of 30 bp or more caused a nonspecific antiviral response in HeLa cells so that it did not cause specific gene silencing (Manche L. et al., Mol. Cell Biol., 12:5238, 1992; Elbashir S.M. et al., Nature, 411:494, 2001). Thus, whether the siRNA complex according to the present invention shows specific and efficient gene silencing when compared with the conventional siRNA structure was analyzed.

In order to confirm whether an antiviral response occurs, each of the siRNA mixture and tsiRNA of Example 2 was bound to PEI and introduced in cells, after which the level of induction of interferon-β (IFN-β) was measured by RT-PCR in the same manner as Example 2. A primer pair used in the RT-PCR was as follows, and poly (I:C)-introduced cells were used as a positive control group causing an antiviral response.
Primer pair for RT-PCR of IFN-β
IFN-β forward: 5'-AGA AGT CTG CAC CTG AAA AGA TAT T-3' (SEQ ID NO: 40)
IFN-β reverse: 5'-TGT ACT CCT TGG CCT TCA GGT AA-3' (SEQ ID NO: 41)

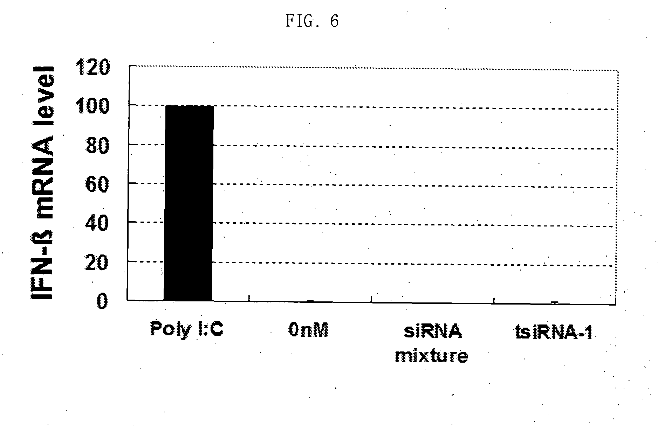

As a result, as can be seen in FIG. 6, the siRNA complex according to the present invention showed less than 1% of the IFN-β mRNA level induced by the positive control group (poly I:C), in the same manner as the case in which the siRNA mixture having the conventional siRNA structure was bonded to PEI and introduced in cells.

Such experimental results indicate that the multiplex siRNA structure of the siRNA complex according to the present invention shows no nonspecific antiviral response, like the conventional siRNA structure, even though it has a length of 30 bp or more.

### Example 7: Preparation of triplex siRNA delivery complex using linker and measurement of gene silencing activity thereof

### 7-1: Preparation of T-tiSurvivin and measurement of gene silencing activity thereof

Using a triplex phosphoramidite compound (Trebler phosphoramidite, Trilink Bio Technology, USA) as shown in FIG. 7, a triplex siRNA delivery complex was constructed.

First, the Trebler phosphoramidite was purchased, and then three strands of a DNA oligonucleotide of SEQ ID NO: 42 was linked to the Trebler phosphoramidite by Genotech Co., Ltd., Korea (indicated by red; 5'->3' direction from the Trebler). Then, an RNA oligonucleotide (SEQ ID NO: 43) having both a region having a nucleotide sequence complementary to the DNA oligonucleotide of SEQ ID NO: 42 and an siSurvivin antisense sequence, and an siSurvivin sense sequence (SEQ ID NO: 44) were annealed at the same time, thereby obtaining T-tiSurvivin as shown in FIG. 7. After that, each of the siSurvivin and long siSurvivin shown in FIG. 8 as a control group and the T-tiSurvivin shown in FIG. 7 was bound to PEI and delivered into HeLa cells, and 24 hours after delivery, the expression level of Survivin mRNA was measured by real-time RT-PCR in the same manner as Example 2. A primer pairs used in the RT-PCR was as follows:
*Survivin*-forward: 5'-GCA CCA CTT CCA GGG TTT AT-3' (SEQ ID NO: 48)
*Survivin*-reverse: 5'-CTC TGG TGC CAC TTT CAA GA-3' (SEQ ID NO: 49)

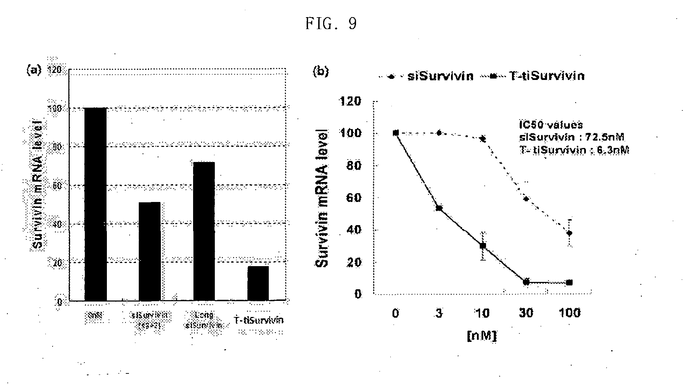

As a result, as can be seen in FIG. 9a, the gene silencing effect of T-tiSurvivin was significantly increased compared to the conventional siRNA or long siSurvivin. Also, the IC₅₀ value was measured. As a result, as can be seen in FIG. 9b, tiSurvivin showed an IC₅₀ value which was about 11 times lower than that of the conventional siRNA structure. This suggests that the inventive triplex siRNA using the linker shows higher gene silencing efficiency than the conventional siRNA structure.

### 7-2: Preparation of T-tiRNA inhibiting the expression of Survivin, cateinin and Integrin and measurement of gene silencing activity thereof

siSurvivin, siIntegrin and siβ-catenin were bound to Trebler phosphoramidite according to the same method as Example 7-1, thereby constructing a triplex siRNA delivery complex as shown in FIG. 10a. Then, each of a T-tiRNA of FIG. 10a and a mixture of siSurvivin, siIntegrin and siβ-catenin of FIG. 8 as a control group was bound to PEI and delivered into HeLa cells. 24 hours after intracellular delivery, the expression levels of Survivin mRNA, Integrin mRNA and β-catenin mRNA were measured by real-time RT-PCR in the same manner as Example 2. Primer pairs used in the RT-PCR were as follows. Herein, the primer pair for Survivin was the same primer pair as used in Example 7-1.
*Integrin*-forward 5'-CGT ATC TGC GGG ATG AAT CT-3' (SEQ ID NO: 54)
*Integrin*-reverse 5'-GGG TTG CAA GCC TGT TGT AT-3' (SEQ ID NO: 55)
β-*catenin*-forward 5'-ATG TCC AGC GTT TGG CTG AA-3' (SEQ ID NO: 56)
β*-catenin-* reverse 5'-TGG TCC TCG TCA TTT AGC AG-3' (SEQ ID NO: 57)
siIntegrin sense: 5'-UGAACUGCACUUCAGAUAU(dTdT)-3' (SEQ ID NO: 58)
siIntegrin antisense: 5'-AUAUCUGAAGUGCAGUUCA(dTdT)-3' (SEQ ID NO: 59)
siβ-catenin sense: 5'-GUAGCUGAUAUUGAUGGACUU-3' (SEQ ID NO: 60)
siβ-catenin antisense: 5'-GUCCAUCAAUAUCAGCUACUU-3' (SEQ ID NO: 61)

As a result, as can be seen in FIG. 10b, the gene silencing effect of T-tiRNA was significantly increased compared to that of the siRNA mixture. This suggests that the inventive triplex siRNA structure using the linker has higher gene silencing efficiency than the conventional siRNA structure.

### 7-3: Preparation of multifunctional MT-tiRNA inhibiting miR-21 and the expression of catenin and Integrin and measurement of gene silencing activity thereof

Anti-miR21, siIntegrin and siβ-catenin were bound to Trebler phosphoramidite in the same manner as Example 7-1, thereby constructing a triplex RNA structure as shown in FIG. 11a. Then, a target sequence for miR-21 was inserted into the Spell and HindIII positions of the 3'-untranslated region of the luciferase mRNA-encoding gene of a pMIR Report-luciferase vector (Ambion), thereby constructing a vector. The inserted target sequences were as follows:
miR-21 target
miR-21 sense:
5'-AATGCACTAGTTCAACATCAGTCTGATAAGCTAGCTCAGCAAGCTTAATGC-3' (SEQ ID NO: 63)
miR-21 antisense:
5'-GCATTAAGCTTGCTGAGCTAGCTTATCAGACTGATGTTGAACTAGTGCATT-3' (SEQ ID NO: 64)

With the vector, each of an MT-tiRNA of FIG. 11a and a mixture of Anti-miR21, siIntegrin and siβ-catenin as a control group was bound to PEI and delivered into HeLa cells, after which the luciferase activity of the cells was measured in the same manner as Example 4.
Anti-miR21: 5'-UCAACAUCAGUCUGAUAAGCUA-3' (SEQ ID NO: 65)

Also, each of an MT-tiRNA of FIG. 11a and a mixture of Anti-miR21, siIntegrin and siβ-catenin as a control group was bound to PEI and delivered into HeLa cells. 24 hours after intracellular delivery, the expression levels of Integrin mRNA and β-catenin mRNA were measured by real-time RT PCR in the same manner as Example 2. The primer pair used in the RT-PCR was the same primer pair as used in Example 7-2.

As a result, as can be seen in FIG. 11b, the MT-tiRNA complex according to the present invention inhibited miR-21, suggesting that it has significantly increased luciferase activity. Namely, it could be seen that the nucleic acid structure complex according to the present invention was a multifunctional structure which could also exhibit a function of inhibiting miRNA.

Also, as can be seen in FIG. 11c, the gene silencing effect of T-tiRNA was significantly increased compared to that of the siRNA mixture. This suggests that the inventive triplex siRNA structure using the linker shows higher gene silencing efficiency than that of the conventional siRNA structure.

### 7-4: Preparation of T-tiAnti-miR21 inhibiting miR-21 and measurement of miR-21 inhibitory activity thereof

In addition, three Anti-miR21s were linked to Trebler phosphoramidite in the same manner as Example 7-1, thereby constructing a triplex RNA structure. Then, the luciferase activity thereof was measured in the same manner as Example 7-3. As a control group, Anti-miR21 was used.

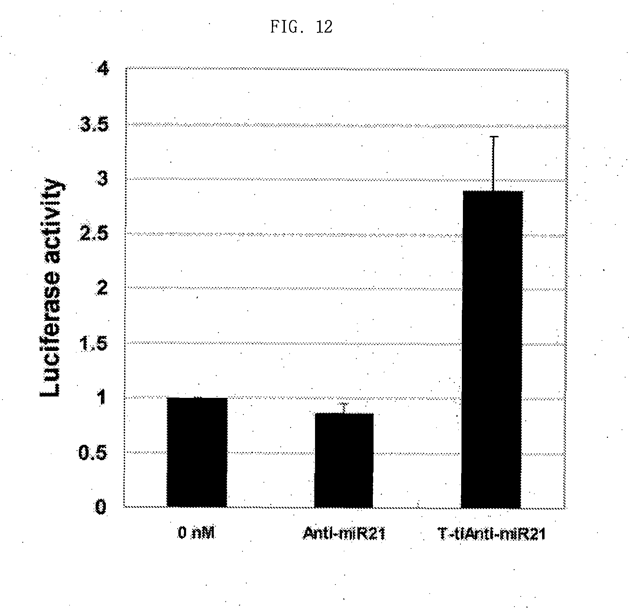

As a result, as can be seen in FIG. 12, in the case in which T-tiAnti-miR21 was introduced, it inhibited miR-21, unlike the case in which the antagomiR Anti-miR21 was introduced, suggesting that the luciferase activity of T-tiAnti-miR21 was significantly increased. Namely, it could be seen that the nucleic acid structure complex according to the present invention inhibited the activity of miRNA at a higher efficiency than the conventional AntagomiR.

### Example 8: Measurement of binding affinity for cationic cell delivery vehicle and intracellular delivery capacity

### 8-1: Comparison of rate of isolation caused by HS

During a process of introducing nucleic acid into cells, the interaction between the nucleic acid and a cationic cell delivery vehicle can be inhibited due to a negatively charged proteoglycan present on the cell surface. Thus, the rate of isolation of the inventive multiplex siRNA complex by the cationic proteoglycan HS (heparan sulfate) that is expressed on the cell surface was analyzed in the following manner. For this purpose, each of the 19-bp siSurvivin of FIG. 8 and the T-tiSurvivin of FIG. 7 was bound to PEI (N/P=5), after which each complex and HS (Sigma/Aldrich) were incubated for 30 minutes while the amount of HS was gradually increased. Then, each of the incubated complexes was electrophoresed on 1% agarose gel, and the amount of siRNA released from each complex was measured. Herein, the measurement was carried out by staining with EtBr and visualization with UV transillumination.

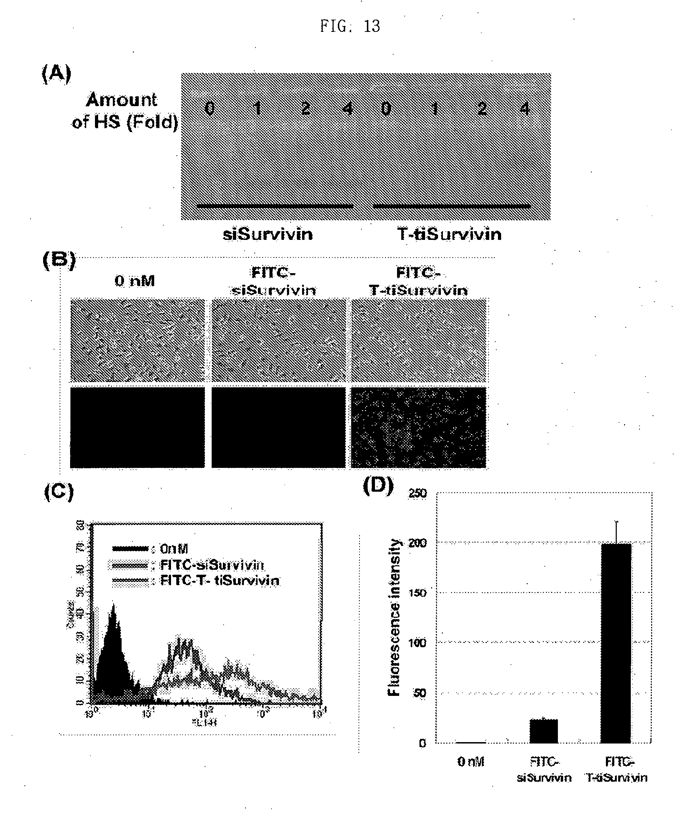

As a result, as can be seen in FIG. 13a, siSurvivin was isolated from PEI by the same amount (1 equivalent (wt/wt)) of HS, whereas the complex according to the present invention was isolated only when it was incubated with a 4-fold increased amount of HS. Such experimental results indicate that the multifunctional RNA structure complex according to the present invention has resistance to the influence of cationic cell surface substances before it is introduced into cells.

### 8-2: Measurement of intracellular delivery capacity using FITC

In order to compare the intracellular delivery capacity between the multifunctional RNA structure (T-tiSurvivin) according to the present invention and the conventional siRNA structure (siSurvivin (FIG. 8)), each of the structures was labeled with FITC in the same manner as Example 3, and then observed with a fluorescence microscope.

As a result, as can be seen in FIG. 13b, siSurvivin was not substantially introduced into cells, but in the case of T-tiSurvivin according to the present invention, scattered green spots could be observed in intracellular regions.

In addition, the fluorescence intensity of the cells introduced with the FITC-labeled RNAs was measured using flow cytometry. The measurement was carried out by binding each of the FITC-labeled T-tiSurvivin and siSurvivin to PEI, transfecting each complex into HeLa cells, collecting the cells 3 hours after transfection, washing the collected cells twice with PBS, and then measuring the fluorescence intensity of the cells using an FACSCalibur system (Becton Dickinson).

As a result, as can be seen in FIG. 13c, the fluorescence intensity of the multifunctional nucleic acid structure according to the present invention was increased compared to the conventional siRNA structure. The fluorescence intensity was quantified and, as a result, as can be seen in FIG. 13d, the average fluorescence intensity was about 10 times different between the structures.

Such results indicate that the multifunctional nucleic acid structure according to the present invention has very excellent intracellular delivery properties compared to the conventional siRNA structure bound to PEI.

### Example 9: Examination of inhibitory effect on cancer cell growth

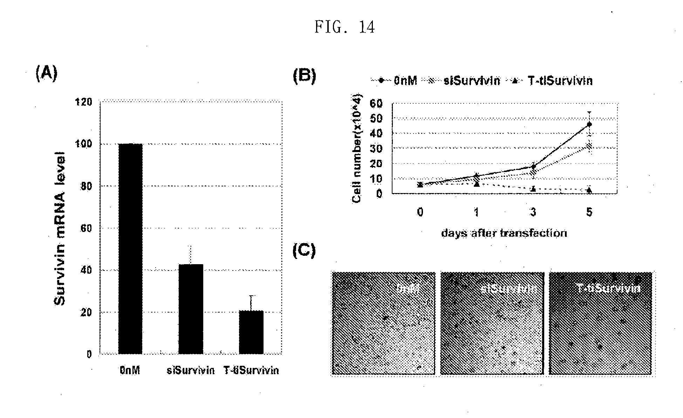

In order to examine whether the complex according to the present invention can be used in an anticancer composition, the expression level of Survivin mRNA was measured in the same manner as Example 7-1. As a result, it was found that T-tiSurvivin according to the present invention significantly inhibited the expression of Survivin in comparison with siSurvivin that is the conventional siRNA structure (FIG. 14a). Then, each of T-tiSurvivin of Fig. 7 and the siSurvivin of FIG. 8 was introduced into liver cancer HepG2 cells (ATCC No. HB-8065), and the degree of inhibition of growth of the cancer cells was measured.

As a result, as can be seen in FIGS. 14b and 14c, introduction of the inventive multifunctional RNA structure T-tiSurvivin bound to PEI showed the effects of inhibiting cancer cell growth and inducing apoptosis in the cancer cells, unlike introduction of siSurvivin and the untreated control group.

Specifically, it was found that the complex according to the present invention has the effect of inhibiting the growth of cancer cells, and thus can be provided as an anticancer composition for treating cancer and inhibiting cancer cell growth.

### Example 10: Examination of induction of immune response

In order to examine whether the multiplex siRNA structure and the nucleic acid structure according to the present invention induce an innate immune response mediated by dsRNA, whether the structures of the present invention express three antiviral immune response genes was examined in comparison with the conventional siRNA structure.

First, each of the T-tiSurvivin of FIG. 7, the siSurvivin of FIG. 8 and the siRNA mixture and tsiRNA of Example 2 was bound to Lipofectamine 2000 and introduced into T98G cells reported to have a very high immune sensitivity to dsRNA treatment, after which the levels of induction of IFIT1, IFN-β and OAS2 were measured by an RT-PCR method in the same manner as Example 2. The primer pairs used in the RT-PCR were as follows, and poly (I:C)-introduced cells were used as a positive control group causing an antiviral response:
Primer pair for RT-PCR of IFIT1
*IFIT1*-Forward 5'-GCC ACA AAA AAT CAC AAG CCA-3' (SEQ ID NO: 66)
*IFIT1*-reverse 5'-CCA TTG TCT GGA TTT AAG CGG-3' (SEQ ID NO: 67)
Primer pair for RT-PCR of IFN-β
IFN-β forward: 5'-AGA AGT CTG CAC CTG AAA AGA TAT T-3' (SEQ ID NO: 40)
IFN-β reverse: 5'-TGT ACT CCT TGG CCT TCA GGT AA-3'(SEQ ID NO: 41)
Primer pair for RT-PCR of OAS2
*OAS2*-forward: 5'-TCA GAA GAG AAG CCA ACG TGA-3' (SEQ ID NO: 68)
OAS2-reverse: 5'-CGG AGA CAG CGA GGG TAA AT-3' (SEQ ID NO: 69)

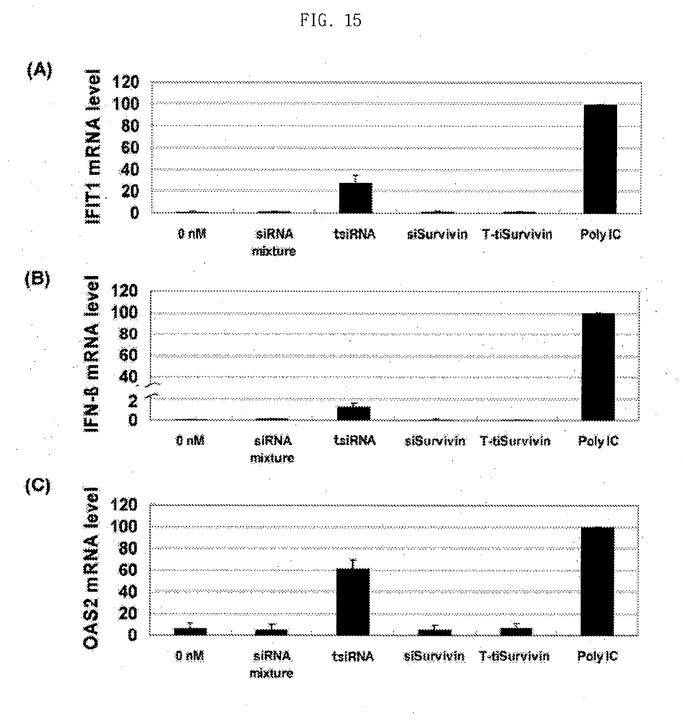

As a result, as can be seen in FIG. 15, unlike the multifunctional nucleic acid structure complex according to the present invention, the multiplex siRNA complex according to the present invention induced the expression of IFIT1 and OAS2, known as viral immune response genes, in T98G cells reported to have a very high immune sensitivity to dsRNA treatment. Namely, it induced a nonspecific immune response. However, the multiplex siRNA complex according to the present invention showed less than 1% of the IFN-β mRNA level induced by the positive control group (poly I:C), similar to Example 2 in which it was introduced into HeLa cells.

Such experimental results suggest that the multifunctional nucleic acid structure complex using a compound having three or more functional groups as a linker is more effective in avoiding the induction of a nonspecific immune response.

### Example 11: Construction of multiplex siRNA complex and multifunctional nucleic acid structure complex, each having chemical modification, and examination of whether the complexes act as substrates for Dicer

It is known that, when long dsRNA is introduced into cells, it is digested by Dicer to produce 21-23-bp siRNAs. Thus, in order to examine whether the multiplex siRNA and multifunctional nucleic acid structures according to the present invention follow mechanisms different from the conventional dsRNAs, the following experiment was carried out.

### 11-1: Examination of whether tsiRNA and T-tiSurvivin act as substrates for Dicer

30 pmol of each of a 27-bp dsRNA, the siLamin and tsiRNA of Example 2, the siSurvivin of FIG. 8 and the T-tiSurvivin of FIG. 7 was allowed to react using a Turbo Dicer siRNA generation Kit (Genlantis) for 12 hours according to the manufacturer's protocol, after which 2 µℓ of a fraction of each reaction product was taken and developed on 10% non-denaturing polyacrylamide gel. The gel was stained with EtBr and visualized by UV transillumination.
27bp dsRNA
Survivin27 Sense: 5'-UCU UAG GAA AGG AGA UCA ACA UUU UCA-3' (SEQ ID NO: 70)
Survivin27 Antisense: 5'-UGA AAA UGU UGA UCU CCU UUC CUA AGA-3'(SEQ ID NO: 71)

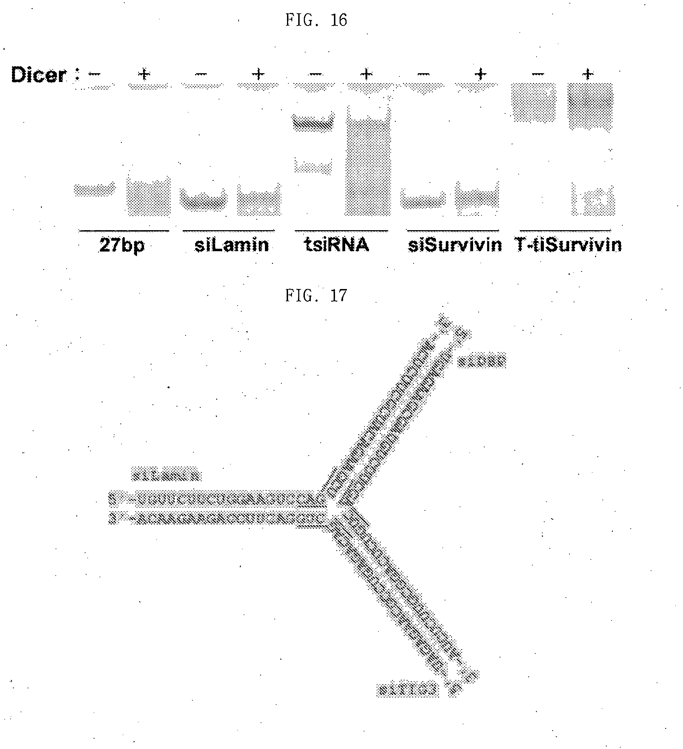

As a result, as can be seen in FIG. 16, unlike the 27-bp dsRNA that was completely digested, the multiplex siRNA or multifunctional nucleic acid structure according to the present invention was not a good substrate for Dicer. However, tsiRNA was partially digested by Dicer. Thus, a multiplex siRNA structure and a multifunctional nucleic acid structure, each having a chemical modification, were constructed and subjected to the following experiments.

### 11-2: Preparation of multiplex siRNA complex and multifunctional nucleic acid structure, each having chemical modification, and examination of whether the structures act as substrates for Dicer

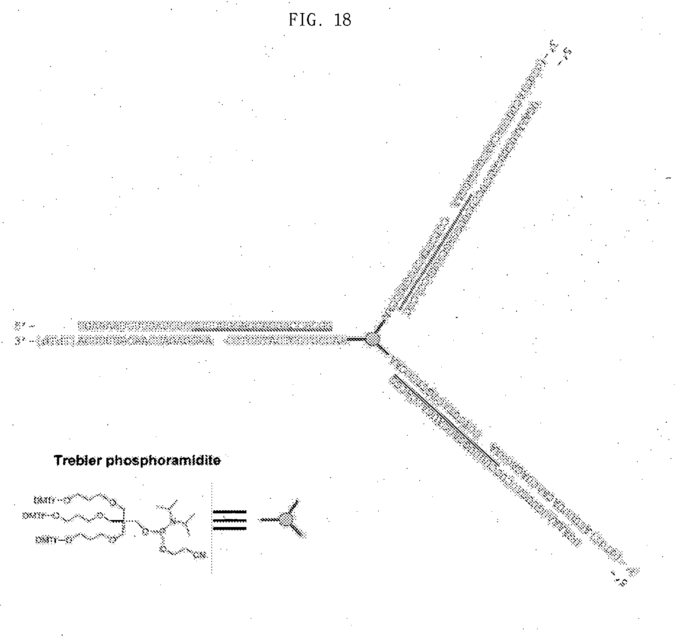

A multiplex siRNA structure (tsiRNA(OMe)) and a multifunctional nucleic acid structure (T-tiSurvivin(OMe)), each having a chemical modification in which the hydroxyl group at the 2-position of the ribose of a nucleotide underlined by gray in one end of each of the tsiRNA of Example 2 and the T-tiSurvivin of FIG. 7 as shown in FIGS. 17 and 18 had been replaced by an -O-methyl group, were prepared, and then subjected to the same experiment as described in Example 11-1.

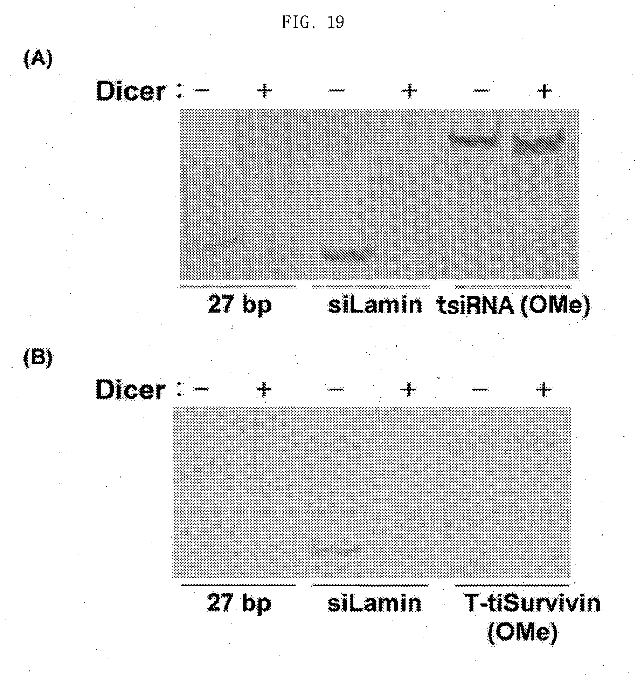

As a result, as can be seen in FIG. 19, both tsiRNA(OMe) and T-tiSurvivin(OMe) did not act as substrates for Dicer.

### 11-3: Examination of gene silencing effects of multiplex siRNA complex and multifunctional nucleic acid structure complex, each having chemical modification

In order to examine whether the multiplex siRNA complex and the multifunctional nucleic acid structure complex, which both had a chemical modification and did not act as substrates for Dicer as described in Example 11-2, have a gene silencing effect, RT-PCR was performed in the same manner as Example 2 and Example 7-1.

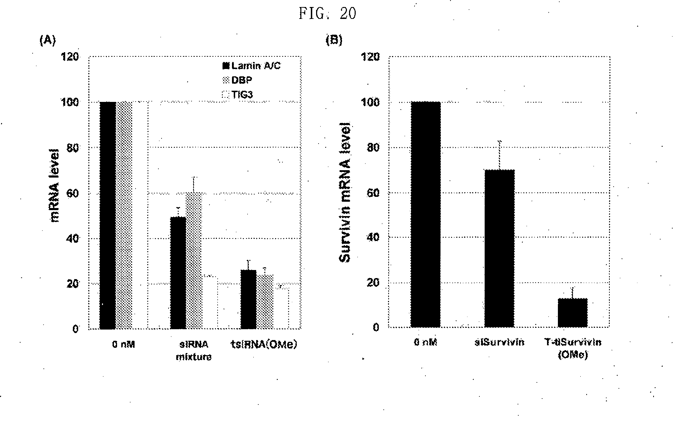

As a result, as can be seen in FIG. 20, both the multiplex siRNA complex and the multifunctional nucleic acid structure complex, each having a chemical modification, have excellent gene silencing effects.

### INDUSTRIAL APPLICABILITY

As described above, the siRNA complex and the multifunctional nucleic acid structure complex according to the present invention have a novel structure which can be chemically synthesized in an easy manner for a conventional shRNA system for inhibiting the expression of a plurality of genes, while they can inhibit the expression of a plurality of genes at the same time at increased efficiency compared to the conventional siRNA. Also, they have high intracellular delivery capacity and can specifically inhibit the expression of target genes without causing a nonspecific antiviral response, and thus are highly useful as siRNA mechanism-mediated therapeutic agents for treating cancer or viral infection. In addition, the multifunctional nucleic acid structure complex can comprise, in addition to siRNAs, functional oligonucleotides, such as miRNA, antagomiR, an antisense oligonucleotide, an aptamer and ribozyme, and thus can perform various functions at the same time.
## Claims

1. A siRNA complex having enhanced intracellular delivery capacity, in which a cationic cell delivery vehicle is bound to a multiplex siRNA structure comprising three siRNAs linked to each other at one end thereof, wherein each of said siRNAs is linked to another siRNA without a linker.

2. The siRNA complex according to claim 1, wherein the three siRNAs are linked to each other at the 3'end of the antisense strand.

3. The siRNA complex according to claim 1 or 2, wherein the siRNA comprises a chemical modification.

4. The siRNA complex according to claim 3, wherein the chemical modification is one in which the hydroxyl group at the 2' position of the ribose of at least one nucleotide included in the siRNA was replaced by any one of a hydrogen atom, a fluorine atom, an -O-alkyl group, an -O-acyl group and an amino group.

5. The siRNA complex according to claim 1, wherein the cationic cell delivery vehicle is a cationic polymer or a cationic lipid.

6. The siRNA complex according to claim 1, wherein the cationic cell delivery vehicle is polyethylenimine or a liposome.

7. Use of the siRNA complex according to any one of claims 1 to 6 in an *in vitro* method of delivering siRNA into cells, wherein said delivering comprises introducing said siRNA complex into cells.

8. A multifunctional nucleic acid structure in which one end of each of three nucleic acid oligonucleotides having a cationic cell delivery vehicle bound to the oligonucleotides, selected from the group consisting of siRNAs, miRNAs, antagomiRs, antisense oligonucleotides, ribozymes and aptamers is linked to a compound having three functional groups, wherein the compound having three functional groups is any one of phosphoramidite compounds, iodoacetyl compounds, maleimide compounds, epoxide compounds, thiol-disulfide compounds, thiolated Ellman's reagent, NHS or sulfo-NHS compounds and isocyanate compounds;
wherein a nucleic acid fragment is linked to each functional group of the compound having three functional groups, and each of the nucleic acid oligonucleotides is linked to the nucleic acid fragment as a complementary bond by introducing a sequence, which can complementarily bind to the nucleic acid fragment, into any one among any one strand of sense or antisense strand of the siRNAs, miRNAs, antagomiRs, antisense oligonucleotides, ribozymes and aptamers.

9. The multifunctional nucleic acid structure according to claim 8, which consists of 3 siRNAs.

10. The multifunctional nucleic acid structure according to claim 8, wherein the nucleic acid oligonucleotide comprises a chemical modification.

11. The multifunctional nucleic acid structure according to claim 10, wherein the chemical modification is one in which the hydroxyl group at the 2' position of the ribose of at least one nucleotide included in the nucleic acid oligonucleotide was replaced by any one of a hydrogen atom, a fluorinse atom, an -O-alkyl group, an -0-acyl group and an amino group.

12. A multifunctional nucleic acid structure complex having enhanced intracellular delivery capacity, in which a cationic cell delivery vehicle is bound to the multifunctional nucleic acid structure of any one claim among claims 8 to 11.

13. The multifunctional nucleic acid structure complex according to claim 12, wherein the cationic cell delivery vehicle is a cationic polymer or cationic lipid.

14. Use of the multifunctional nucleic acid structure complex according to claim 12 in an *in vitro* method of delivering a multifunctional nucleic acid structure into cells, wherein said delivering comprises introducing said multifunctional nucleic acid structure complex into cells.

15. A method of preparing a multifunctional nucleic acid structure complex having enhanced intracellular delivery capacity, the method comprising the steps of:
linking one end of each of nucleic acid oligonucleotides, selected from the group consisting of siRNAs, miRNAs, antagomiRs, antisense oligonucleotides, ribozymes and aptamers, to a compound having three functional groups, wherein the compound having three functional groups is anyone of phosphoramidite compounds, iodoacetyl compounds, maleimide compounds, epoxide compounds, thiol-disulfide compounds, thiolated Ellman's reagent, NHS or sulfo-NHS compounds and isocyanate compounds, thereby preparing a multi-functional nucleic acid structure, wherein a nucleic acid fragment is linked to each functional group of the compound having three functional groups, and each of the nucleic acid oligonucleotides is linked to the nucleic acid fragment as a complementary bond by introducing a sequence, which can complementarily bind to the nucleic acid fragment, into any one among any one strand of sense or antisense strand of the siRNAs, miRNAs, antagomiRs, antisense oligonucleotides, ribozymes and aptamers; and binding a cationic cell delivery vehicle to the multifunctional nucleic acid structure by charge-charge interaction.

## Patentansprüche

1. siRNA-Komplex, der eine verstärkte intrazelluläre Abgabekapazität hat, in dem ein kationisches Zellabgabevehikel an eine Multiplex-siRNA-Struktur, die drei siRNAs, die an einem Ende davon miteinander verknüpft sind, umfasst, gebunden ist, wobei jede der siRNAs ohne einen Linker mit einer anderen siRNA verknüpft ist.

2. siRNA-Komplex gemäß Anspruch 1, wobei die drei siRNAs am 3'-Ende des Antisense-Strangs miteinander verknüpft sind.

3. siRNA-Komplex gemäß Anspruch 1 oder 2, wobei die siRNA eine chemische Modifikation umfasst.

4. siRNA-Komplex gemäß Anspruch 3, wobei die chemische Modifikation eine ist, bei der die Hydroxylgruppe an der 2'-Position der Ribose von wenigstens einem Nucleotid, das in der siRNA enthalten ist, durch ein(e) beliebige(s) von einem Wasserstoffatom, einem Fluoratom, einer -O-Alkylgruppe, einer -O-Acylgruppe und einer Aminogruppe, ersetzt worden ist.

5. siRNA-Komplex gemäß Anspruch 1, wobei das kationische Zellabgabevehikel ein kationisches Polymer oder ein kationisches Lipid ist.

6. siRNA-Komplex gemäß Anspruch 1, wobei das kationische Zellabgabevehikel Polyethylenimin oder ein Liposom ist.

7. Verwendung des siRNA-Komplexes gemäß einem der Ansprüche 1 bis 6 in einem In-vitro-Verfahren zur Abgabe von siRNA in Zellen, wobei die Abgabe Einführen des siRNA-Komplexes in Zellen umfasst.

8. Multifunktionelle Nucleinsäurestruktur, in welcher ein Ende jedes von drei Nucleinsäure-Oligonucleotiden, die ein kationisches Zellabgabevehikel an die Oligonucleotide gebunden haben, ausgewählt aus der Gruppe, bestehend aus siRNAs, miRNAs, antagomiRs, Antisense-Oligonucleotiden, Ribozymen und Aptameren, mit einer Verbindung, die drei funktionelle Gruppen hat, verknüpft ist, wobei die Verbindung, die drei funktionelle Gruppen hat, eine beliebige von Phosphoramiditverbindungen, Iodacetylverbindungen, Maleimidverbindungen, Epoxidverbindungen, Thiol-Disulfid-Verbindungen, thioliertem Ellmans Reagenz, NHS- oder Sulfo-NHS-Verbindungen und Isocyanatverbindungen ist;
worin ein Nucleinsäurefragment mit jeder funktionellen Gruppe der Verbindung, die drei funktionelle Gruppen hat, verknüpft ist und jedes der Nucleinsäure-Oligonucleotide mit dem Nucleinsäurefragment als komplementäre Bindung verknüpft ist, und zwar durch Einführen einer Sequenz, welche komplementär an das Nucleinsäurefragment binden kann, in einen beliebigen aus einem Sense-Strang oder Antisense-Strang der siRNAs, miRNAs, antagomiRs, Antisense-Oligonucleotide, Ribozyme und Aptamere.

9. Multifunktionelle Nucleinsäurestruktur gemäß Anspruch 8, die aus drei siRNAs besteht.

10. Multifunktionelle Nucleinsäurestruktur gemäß Anspruch 8, wobei das Nucleinsäure-Oligonucleotid eine chemische Modifikation umfasst.

11. Multifunktionelle Nucleinsäurestruktur gemäß Anspruch 10, wobei die chemische Modifikation eine ist, bei der die Hydroxylgruppe an der 2'-Position der Ribose wenigstens eines Nucleotids, das in dem Nucleinsäure-Oligonucleotid enthalten ist, durch eine(s) von einem Wasserstoffatom, einem Fluoratom, einer -O-Alkylgruppe, einer -O-Acylgruppe und einer Aminogruppe ersetzt worden ist.

12. Multifunktioneller Nucleinsäurestrukturkomplex, der eine verstärkte intrazelluläre Abgabekapazität hat, in dem ein kationisches Zellabgabevehikel an die multifunktionelle Nucleinsäurestruktur gemäß einem der Ansprüche 8 bis 11 gebunden ist.

13. Multifunktioneller Nucleinsäurestrukturkomplex gemäß Anspruch 12, wobei das kationische Zellabgabevehikel ein kationisches Polymer oder ein kationisches Lipid ist.

14. Verwendung des multifunktionellen Nucleinsäurestrukturkomplexes gemäß Anspruch 12 in einem In-vitro-Verfahren zur Abgabe einer multifunktionellen Nucleinsäurestruktur in Zellen, wobei die Abgabe Einführen des multifunktionellen Nucleinsäurestrukturkomplexes in Zellen umfasst.

15. Verfahren zur Herstellung eines multifunktionellen Nucleinsäurestrukturkomplexes, der verstärkte intrazelluläre Abgabekapazität hat, wobei das Verfahren die folgenden Schritte umfasst:
Verknüpfen von einem Ende jedes der Nucleinsäure-Oligonucleotide, ausgewählt aus der Gruppe, bestehend aus siRNAs, miRNAs, antagomiRs, Antisense-Oligonucleotiden, Ribozymen und Aptameren, mit einer Verbindung, die drei funktionelle Gruppen hat, wobei die Verbindung, die drei funktionelle Gruppen hat, eine beliebige von Phosphoramiditverbindungen, Iodacetylverbindungen, Maleimidverbindungen, Epoxidverbindungen, Thioldisulfidverbindungen, thioliertem Ellmans Reagenz, NHS- oder Sulfo-NHS-Verbindungen und Isocyanatverbindungen ist, dadurch Herstellen einer multifunktionellen Nucleinsäurestruktur, in der ein Nucleinsäurefragment mit jeder funktionellen Gruppe der Verbindung, die drei funktionelle Gruppen hat, verknüpft ist und jedes der Nucleinsäure-Oligonucleotide mit dem Nucleinsäurefragment als eine komplementäre Bindung verknüpft wird, indem eine Sequenz, welche komplementär an das Nucleinsäurefragment binden kann, in einen beliebigen Strang von einem Sense-Strang oder Antisense-Strang der siRNAs, miRNAs, antagomiRs, Antisense-Oligonucleotiden, Ribozymen und Aptameren eingeführt wird, und Binden eines kationischen Zellabgabevehikels an die multifunktionelle Nucleinsäurestruktur durch Ladungs-Ladungs-Wechselwirkung.

## Revendications

1. Complexe de type ARNsi ayant une capacité d'administration intracellulaire améliorée, dans lequel un véhicule d'administration cellulaire de type cationique est lié à une structure ARNsi multiplex comprenant trois ARNsi liés les uns aux autres par une de leurs extrémités, chacun desdits ARNsi étant lié à un autre ARNsi sans lieur.

2. Complexe de type ARNsi selon la revendication 1, dans lequel les trois ARNsi sont liés les uns aux autres à l'extrémité 3' du brin antisens.

3. Complexe de type ARNsi selon la revendication 1 ou 2, dans lequel l'ARNsi comprend une modification chimique.

4. Complexe de type ARNsi selon la revendication 3, dans lequel la modification chimique est du type dans lequel le groupe hydroxyle à la position 2' du ribose d'au moins un nucléotide contenu dans l'ARNsi a été remplacé par un atome d'hydrogène, ou un atome de fluor, ou un groupe -O-alkyle, ou un groupe -O-acyle, ou un groupe amino.

5. Complexe de type ARNsi selon la revendication 1, dans lequel le véhicule d'administration cellulaire de type cationique est un polymère cationique ou un lipide cationique.

6. Complexe de type ARNsi selon la revendication 1, dans lequel le véhicule d'administration cellulaire de type cationique est un polyéthylèneimine ou un liposome.

7. Utilisation du complexe de type ARNsi selon l'une quelconque des revendications 1 à 6 dans un procédé *in vitro* d'administration d'ARNsi dans des cellules, ladite administration comprenant l'introduction dudit complexe de type ARNsi dans des cellules.

8. Structure d'acide nucléique multifonctionnelle dans laquelle une extrémité de chacun des trois oligonucléotides d'acide nucléique auquel est lié un véhicule d'administration cellulaire de type cationique, lesdits oligonucléotides étant choisis dans le groupe constitué par les ARNsi, les miARN, les antagomiR, les oligonucléotides antisens, les ribozymes et les aptamères, est liée à un composé ayant trois groupes fonctionnels, le composé ayant trois groupes fonctionnels étant un composé quelconque parmi les composés phosphoramidite, les composés iodoacétyle, les composés maléimide, les composés époxyde, les composés thiol-disulfure, le réactif d'Ellman thiolé, les composés NHS ou sulfo-NHS et les composés isocyanate ;
dans laquelle un fragment d'acide nucléique est lié à chaque groupe fonctionnel du composé ayant trois groupes fonctionnels, et chacun des oligonucléotides d'acide nucléique est lié au fragment d'acide nucléique sous forme de liaison complémentaire par introduction d'une séquence, qui peut se lier de manière complémentaire au fragment d'acide nucléique, dans l'un quelconque d'un brin quelconque sens ou antisens des ARNsi, miARN, atagomiR, oligonucléotides antisens, ribozymes et aptamères.

9. Structure d'acide nucléique multifonctionnelle selon la revendication 8, qui consiste en 3 ARNsi.

10. Structure d'acide nucléique multifonctionnelle selon la revendication 8, dans laquelle l'oligonucléotide d'acide nucléique comprend une modification chimique.

11. Structure d'acide nucléique multifonctionnelle selon la revendication 10, dans laquelle la modification chimique est du type dans lequel le groupe hydroxyle à la position 2' du ribose d'au moins un nucléotide contenu dans l'oligonucléotide d'acide nucléique a été remplacé par un atome d'hydrogène, ou un atome de fluor, ou un groupe -O-alkyle, ou un groupe -O-acyle, ou un groupe amino.

12. Complexe de type structure d'acide nucléique multifonctionnelle ayant une capacité d'administration intracellulaire améliorée, dans lequel un véhicule d'administration cellulaire de type cationique est lié à la structure d'acide nucléique multifonctionnelle selon l'une quelconque des revendications 8 à 11.

13. Complexe de type structure d'acide nucléique multifonctionnelle selon la revendication 12, dans lequel le véhicule d'administration cellulaire de type cationique est un polymère cationique ou un lipide cationique.

14. Utilisation du complexe de type structure d'acide nucléique multifonctionnelle selon la revendication 12 dans un procédé *in vitro* d'administration d'une structure d'acide nucléique multifonctionnelle dans des cellules, ladite administration comprenant l'introduction dudit complexe de type structure d'acide nucléique multifonctionnelle dans des cellules.

15. Procédé de préparation d'un complexe de type structure d'acide nucléique multifonctionnelle ayant une capacité d'administration intracellulaire améliorée, le procédé comprenant les étapes de :
liaison d'une extrémité de chacun des oligonucléotides d'acide nucléique, choisis dans le groupe constitué par les ARNsi, les miARN, les antagomiR, les oligonucléotides antisens, les ribozymes et les aptamères, à un composé ayant trois groupes fonctionnels, le composé ayant trois groupes fonctionnels étant un composé quelconque parmi les composés phosphoramidite, les composés iodoacétyle, les composés maléimide, les composés époxyde, les composés thiol-disulfure, le réactif d'Ellman thiolé, les composés NHS ou sulfo-NHS et les composés isocyanate, pour préparer ainsi une structure d'acide nucléique multifonctionnelle, dans laquelle un fragment d'acide nucléique est lié à chaque groupe fonctionnel du composé ayant trois groupes fonctionnels, et chacun des oligonucléotides d'acide nucléique est lié au fragment d'acide nucléique sous forme de liaison complémentaire par introduction d'une séquence, qui peut se lier de manière complémentaire au fragment d'acide nucléique, dans l'un quelconque d'un brin quelconque sens ou antisens des ARNsi, miARN, antagomiR, oligonucléotides antisens, ribozymes et aptamères ; et liaison d'un véhicule d'administration cellulaire de type cationique à la structure d'acide nucléique multifonctionnelle par interaction charge-charge.
